# EUROPEAN PATENT SPECIFICATION

(11) **EP 2 245 181 B1**
(45) Date of publication and mention of the grant of the patent: **12.10.2011**
(21) Application number: 09709335.5
(22) Date of filing: 03.02.2009
(51) Int. Cl.: C12Q 1/48, G01N 33/573

(54) **SELECTIVITY PROFILING OF PI3K INTERACTING MOLECULES AGAINST MULTIPLE TARGETS**
SELEKTIVITÄTSPROFILERSTELLUNG VON MIT PI3K WECHSELWIRKENDEN MOLEKÜLEN GEGEN MEHRERE ZIELE
DÉTERMINATION DE PROFIL DE SÉLECTIVITÉ DE MOLÉCULES INTERAGISSANT AVEC LA PI3K DIRIGÉES CONTRE DES CIBLES MULTIPLES

(30) Priority: 04.02.2008 EP 08002053
(43) Date of publication of application: 03.11.2010
(73) Proprietor: CELLZOME AG, 69117 Heidelberg (DE)
(72) Inventor: CANSFIELD, Andrew David, Cambridge CB22 7QT (GB); BERGAMINI MOORE, Giovanna, 69121 Heidelberg (DE); NEUBAUER, Gitte, 68259 Mannheim (DE)
(74) Representative: Lahrtz, Fritz
(86) International application number: PCT/EP2009/000692
(87) International publication number: WO 2009/098021

(56) References cited:
- WO-A-95/10628
- WO-A-2006/051270
- WALKER EDWARD H ET AL: "Structural determinants of phosphoinositide 3-kinase inhibition by wortmannin, LY294002, quercetin, myricetin, and staurosporine" MOLECULAR CELL, CELL PRESS, CAMBRIDGE, MA, US, vol. 6, no. 4, 1 October 2000 (2000-10-01), pages 909-919, XP002214228 ISSN: 1097-2765

## Description

The present invention relates to methods for the identification and characterization of PI3K interacting molecules and for the purification of PI3K using 3-(2-{2-[2-(2-amino-ethoxy)-ethoxy]-ethoxy}-ethoxy)-N-[5-(4-chloro-3-methane sulfonyl-phenyl)-4-methyl-thiazol-2yl]propionamide as a ligand for PI3K. Furthermore, the present invention relates to pharmaceutical compositions comprising said interacting molecules e.g. for the treatment of cancer, metabolic diseases or autoimmune/inflammatory disorders.

Kinases catalyze the phosphorylation of proteins, lipids, sugars, nucleosides and other cellular metabolites and play key roles in all aspects of eukryotic cell physiology. Especially, protein kinases and lipid kinases participate in the signaling events which control the activation, growth, differentiation and survival of cells in response to extracellular mediators or stimuli such as growth factors, cytokines or chemokines. In general, protein kinases are classified in two groups, those that preferentially phosphorylate tyrosine residues and those that preferentially phosphorylate serine and/or threonine residues.

Inappropriately high protein kinase activity is involved in many diseases including cancer, metabolic diseases and autoimmune/inflammatory disorders. This can be caused either directly or indirectly by the failure of control mechanisms due to mutation, overexpression or inappropriate activation of the enzyme. In all of these instances, selective inhibition of the kinase is expected to have a beneficial effect.

One group of lipid kinases that has become a recent focus of drug discovery is the phosphoinositide 3-kinase (PI3K) family. Members of the PI3K family are lipid kinases that catalyse the transfer of the gamma-phosphate from ATP to the 3'-hydroxyl group of phophatidyiinositoi and its derivatives, collectively called phosphoinositides. Eight members (isoforms) of the PI3K family have been isolated from mammalian cells so far and grouped into three classes according to their primary structure and substrate specificity (class IA: PI3K alpha, beta and delta; class IB: PI3K gamma; class II: PI3KC2 alpha, beta and gamma; class III: Vps34 yeast homologue) (Fruman et al., 1998. Phosphoinositide kinases. Annual Review Biochemistry 67, 481-507; Cantley, L.C., 2002, Science 296, 1655-1657).

Mammalian cells are known to express three isoforms of the catalytic subunit of P13K IA class (p110 alpha, p110 beta and p110 delta, synonym "PI3K delta"). Class IB contains only one member (catalytic subunit) which has been named p110gamma or PI3K gamma. In addition to its lipid kinase activity PI3K gamma exhibits also a serine/threonine protein kinase acitivity as demonstrated by autophosphorylation.

The study of genetically manipulated mice in which the genes encoding PI3K gamma or delta were deleted give important information about the physiological function of these kinases and their potential utility as drug targets. Mice lacking PI3K gamma or delta are viable and exhibit distinctive phenotypes suggesting several potential therapeutic indications. PI3K gamma appears to be a major mediator of the innate immune system. For example, PI3K gamma deficient macrophages and neutrophilic granulocytes display an impaired ability to infiltrate the inflamed peritoneum. Mast cells represent another cell type affected in PI3K gamma deficient mice. The phenotype of mice lacking PI3K delta is characterized by an impairment of lymphocyte functions and point to a dominant function in the control of the adaptive immune response (Wetzker and Rommel, Current Pharmaceutical Design, 2004, 10,1915-1922).

In contrast to the widely expressed PI3K alpha and beta isoforms the hematopoietic specific isoforms PI3K gamma and delta suggest important therapeutic indications. Both isoforms appear as ideal targets for the treatment of autoimune/inflammatory diseases mediated by hyperactive phagocytes, mast cells, B-and T-lymphocytes (e.g. rheumatoid arthritis, asthma or allergic reactions). In order to avoid unwanted side effects highly isoform selective inhibitors are necessary (Ohashi and Woodgett 2005, Nature Medicine 11, 924-925).

Members of the phosphatidylinositol kinase-related kinase (PIKK) family are high molecular mass kinases involved in cell cycle progression, DNA recombination, and the detection of DNA damage. The human ATM gene, which is defective in cells of patients with ataxia-telangiectasia and is involved in detection and response of cells to damaged DNA, is a member of this family. Another is mTOR (synonym FRAP), which is involved in a rapamycin-sensitive pathway leading to G1 cell cycle progression (Shilo, 2003. Nature Reviews Cancer 3, 155-168).

One prerequisite for the identification and characterization of PI3K inhibitors is the provision of suitable assays, preferably physiological forms of the protein target. In the art, several strategies have been proposed to address this issue.

Conventionally, PI3K lipid kinase activity can be measured using purified or recombinant enzyme in a solution-based assay with phopholipid vesicles. The reaction is terminated by the addition of acidified organic solvents and subsequent phase separation by extraction or thin layer chromatography analysis (Carpenter et al., 1990, J. Biol. Chem. 265, 19704-19711).

Another assay described in the art is based on the phosphate transfer from radiolabeled ATP to phosphatidylinositol immobilized on plates. This assay type also uses recombinant PI3K gamma enzyme but can be performed in a high throughput mode (Fuchikami et al., 2002, J. Biomol. Screening 7, 441-450).

Yet another biochemical screening assay is based on a competitive fluorescence polarization (FP) format using fluorophore-labeled phosphoinositide (Drees et al., 2003, Comb. Chem. High Throughput Screening 6, 321-330).

Finally, a cell-based Akt-EGFP redistribution assay was reported based on fluorescence microscopic imaging and automated image analysis. To this end Chinese Hamster Ovary (CHO) cells were stably transfected with the human insulin receptor and an Akt1-enhanced green fluorescent protein (EGFP) fusion construct. After stimulation with insulin-like growth factor-1 (IGF-1) PI3K was activated and the Akt1-EGFP protein was recruited to the cell membrane. The validation of the redistribution assay with P13K isoform selective inhibitors showed that PI3K alpha is the main isoform activated in CHO host cells after IGF-1 stimulation (Wolff et al., Comb. Chem. High Throughput Screen. 9, 339-350).

Another, although not in all instances necessary prerequisite for the identification of selective kinase inhibitors is a method that allows to determine the target selectivity of these molecules. For example, it can be intended to provide molecules that bind to and inhibit a particular drug target but do not interact with a closely related target, inhibition of which could lead to side effects. Conventionally large panels of individual enzyme assays are used to assess the inhibitory effect of a compound for kinases (Knight et al., 2004. Bioorganic and Medicinal Chemistry 12, 4749-4759; Knight et al., 2006, Cell 125, 733-747). More recently, kinases or kinase domains displayed on bacteriophages have been employed to assess the ability of a given compound to interact with a large set of kinases (Karaman et al., 2008. Nature Biotechnology 26, 127-132). In addition, chemical proteomics methods have been described which allow the profiling of kinase inhibitors against the proteome (WO 2006/134056; Bantscheff et al., 2007. Nature Biotechnology 25, 1035-1044; Patricelly et al., 2007. Biochemistry 46, 350-358; Gharbi et al., 2007. Biochem. J. 404, 15-21; WO2008/015013).

In view of the above, there is a need for providing effective methods for the identification and selectivity profiling of PI3K interacting compounds as well as for methods for the purification of PI3K.

To comply with this need, the invention provides in a first aspect a method for the identification of a PI3K interacting compound, comprising the steps of
a) providing a protein preparation containing PI3K,
b) contacting the protein preparation with 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide or a salt thereof immobilized on a solid support under conditions allowing the formation of a 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide - PI3K complex,
c) incubating the 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide - PI3K complex with a given compound,
d) determining whether the compound is able to separate PI3K from the immobilized 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide, and
e) determining whether the compound is able to separate also ATM, ATR, DNAPK and/or mTOR from the immobilized 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide.

In a second aspect, the present invention relates to a method for the identification of a PI3K interacting compound, comprising the steps of
a) providing a protein preparation containing PI3K,
b) contacting the protein preparation with 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide or a salt thereof immobilized on a solid support and with a given compound under conditions allowing the formation of a 3-(2-{2-[2-(2-amino-ethoxy)-ethoxy]-ethoxy}-ethoxy)-N-[3-(4-chloro-3-methanesulfonyl-phenyl)-4-methyl-thiazol-2yl]-propionamideI3K complex,
c) detecting the 3-(2-{2-[2-(2-amino-ethoxy)-ethoxy]-ethoxy}-ethoxy)-N-[S-(4-chloro-3-methanesulfonyl-phenyl)-4-methyl-thiazol-2yl]-propionamide - PI3K complex formed in step b), and
d) detecting whether also a complex between 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide and ATM, ATR, DNAPK and or mTOR has been formed in step b).

In a third aspect, the invention provides a method for the identification of a PI3K interacting compound, comprising the steps of:
a) providing two aliquots of a protein preparation containing PI3K,
b) contacting one aliquot with the 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide or a salt thereof immobilized on a solid support under conditions allowing the formation of a 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide- PI3K complex,
c) contacting the other aliquot with the 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide or a salt thereof immobilized on a solid support and with a given compound under conditions allowing the formation of a 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide-PI3K complex,
d) determining the amount of the 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide- PI3K complex formed in steps b) and c), and
e) determining whether also a complex between 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide and ATM, ATR, DNAPK and or mTOR has been formed in steps b) and c).

In a fourth aspect, the invention relates to a method for the identification of a PI3K interacting compound, comprising the steps of:
a) providing two aliquots comprising each at least one cell containing PI3K,
b) incubating one aliquot with a given compound,
c) harvesting the cells of each aliquot,
d) lysing the cells in order to obtain protein preparations,
e) contacting the protein preparations with the 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide immobilized on a solid support under conditions allowing the formation of a 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide- PI3K complex, and
f) determining the amount of the 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide - PI3K complex formed in each aliquot in step e), and
g) determining whether also a complex between 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide and ATM, ATR, DNAPK and or mTOR has been formed in step e).

In the context of the present invention, it has been surprisingly found that phenylthiazole ligand 1 (3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide or a salt thereof) is a PI3K ligand and a ligand of other members of the PIKK family, namely ATM, ATR, DNAPK and mTOR (FRAP). This enables the use of phenylthiazole ligand 1 in screening assays, e.g. in competitive screening assays as well as in methods for the purification of PI3K.

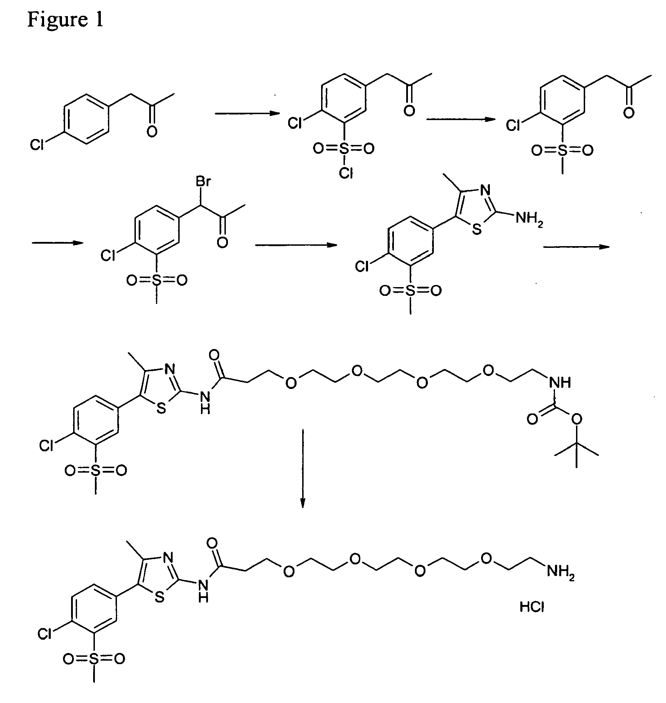

The structure of 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide is also given in Figure 1. This compound is a substituted thiazole (3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide) which according to Figure 1 has hydrochloride as the anion in liquid solution. However, further counter ions are also envisaged in the context of the present invention. The 3-(2-{2-[2-(2-amino-ethoxy)-ethoxy]-ethoxy}-ethoxy)-N-[5-(4-chloro-3-methanesulfonylphenyl)-4-methyl-thiazol-2yl]-propionamide can be covalently coupled to a suitable solid support material via the primary amino group and be used for the isolation of binding proteins. The synthesis of 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide is described in Example 1. According to the invention the expression "3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide or a salt thereof" also includes compounds comprising the identical core but which have another linker, preferably coupled to the nitrogen not being part of the cyclic structures, for linkage to the solid support. Typically linkers have backbone of 8, 9 or 10 atoms. The linkers may contain either a carboxy-, hydroxy or amino-active group.

Therefore, in a preferred embodiment, the expression "3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide or a salt thereof" also includes compounds having the same N-[5-(4-chloro-3-methanesulfonyl-phenyl)-4-methyl-thiazol-2yl]-propionamide core but comprise another linker at the N-atom, e.g. a C1-C8 alkylcarbonyl or a C1-C8 alkylaminocarbonyl, either of which being optionally substituted by halogen, hydroxy, amino, C1-C8-alkylamino, C1-C8-alkoxycarbonyl, C1-C8-alkoxy optionally substituted by hydroxyl or C1-C8-alkyl optionally substituted by hydroxyl or halogen. Furthermore, this expression also includes compounds as described above which have instead of the 4-chloro residue another halogen, e.g. bromide or which are further substituted at the phenyl ring, e.g. by halogen. Furthermore, instead of the methane sulfonyl group, also another group like a hydroxyl, carboxyl or C1-C8 alkyl group, optionally substituted by halogen, may be present.

In an especially preferred embodiment, compounds falling under the expression "3-(2-{2-[2-(2-amino-ethoxy)-ethoxy]-ethoxy}-ethoxy)-N-[5-(4-chloro-3-methanesulfonylphenyl)-4-methyl-thiazol-2yl]-propionamide or a salt thereof" are selected from the group consisting of 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide hydrochloride, 3-(2-{2-[2-(2-amino-ethoxy)-ethoxy]-ethoxy}-ethoxy)-N-[5-(4-chloro-3-methanesulfonylphenyl)-4-methyl-thiazol-2yl]-propionamide, and compounds with the same N-[5-(4-chloro-3-methanesulfonyl-phenyl)-4-methyl-thiazol-2yl]-propionamide core which are only further substituted at the N- atom by C1-C8 alkylcarbonyl or C1-C8 alkylaminocarbonyl, either of which being optionally substituted by halogen, hydroxy, amino, C1-C8-alkylamino, C1-C8-alkoxycarbonyl, C1-C8-alkoxy optionally substituted by hydroxyl or C1-C8-alkyl optionally substituted by hydroxyl or halogen

According to the present invention "PI3K" comprises all members of the PI3K family comprising class IA (e.g. PI3K alpha, beta and delta), class IB (e.g. PI3K gamma), class II (e.g. PI3KC2 alpha, beta and gamma) and class III (e.g. Vps34 yeast homologue).

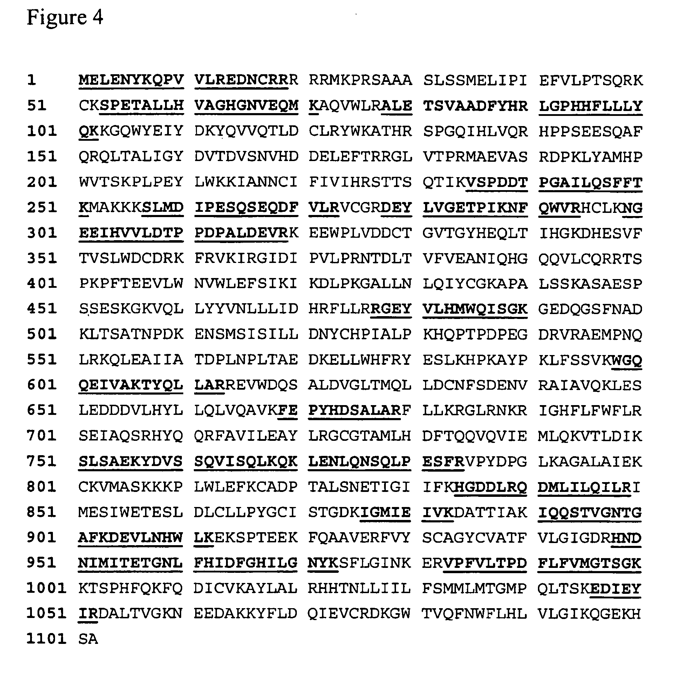

The sequence of human PI3K gamma (the so far only known member of class IB) is given in Figure 4.

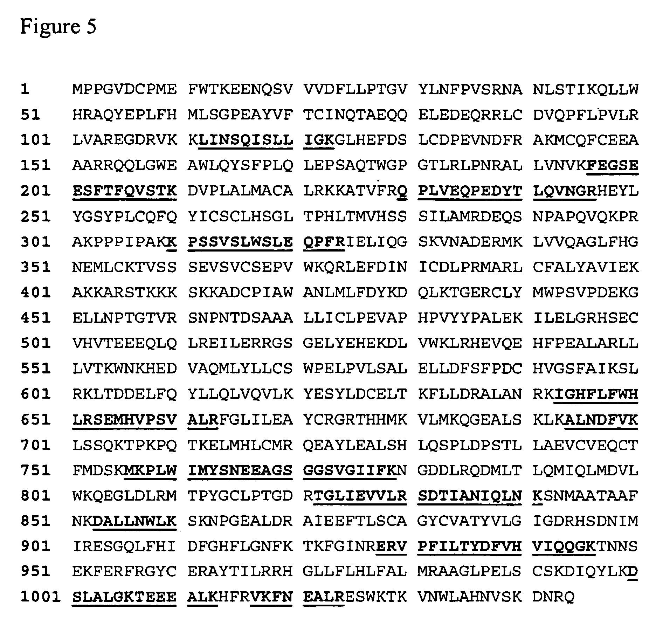

The sequence of human PI3K delta (a member of class IA) is given in Figure 5.

According to the present invention, the expression "PI3K" relates to both human and other proteins of this family. The expression especially includes functionally active derivatives thereof, or functionally active fragments thereof, or a homologues thereof, or variants encoded by a nucleic acid that hybridizes to the nucleic acid encoding said protein under low stringency conditions. Preferably, these low stringency conditions include hybridization in a buffer comprising 35% formamide, 5X SSC, 50 mM Tris-HCl (pH 7.5), 5 mM EDTA, 0.02% PVP, 0.02% BSA, 100 ug/ml denatured salmon sperm DNA, and 10% (wt/vol) dextran sulfate for 18-20 hours at 40°C, washing in a buffer consisting of 2X SSC, 25 mM Tris-HCl (pH 7.4), 5 mM EDTA, and 0.1% SDS for 1-5 hours at 55°C, and washing in a buffer consisting of 2X SSC, 25 mM Tris-HCl (pH 7.4) 5 mM EDTA, and 0.1 % SDS for 1.5 hours at 60°C.

According to the present invention, "ATM" means Ataxia Telangiectasia Mutated protein. The ATM protein is a member of the phosphatidylinositol-3 kinase family of proteins that respond to DNA damage by phosphorylating key substrates involved in DNA repair and/or cell cycle control (Shilo, 2003. Nature Reviews Cancer 3, 155-168).

According to the present invention, "ATR" means Ataxia Telangiectasia and RAD3-Related protein (synonym FRAP-related protein 1, FRP1).

According to the present invention, "DNAPK" means DNA-dependent protein kinase. The PRKDC gene encodes the catalytic subunit of a nuclear DNA-dependent serine/threonine protein kinase (DNA-PK). The second component is the autoimmune antigen Ku (152690), which is encoded by the G22P1 gene on chromosome 22q. On its own, the catalytic subunit of DNA-PK is inactive and relies on the G22P1 component to direct it to the DNA and trigger its kinase activity; PRKDC must be bound to DNA to express its catalytic properties.

According to the present invention, "mTOR" means mammalian target of rapamycin (mTOR, also known as FRAP or RAFT1) (Tsang et al., 2007, Drug Discovery Today 12, 112-124). The mTOR protein is a large kinase of 289 kDA which occurs in all eukaryotic organisms sequenced so far. The sequence of the carboxy-terminal "phosphatidylinositol 3-kinase (PI3K)-related kinase" (PIKK) domain is highly conserved between species and exhibits serine and threonine kinase activity but no detectable lipid kinase activity. According to the present invention, the expressions "ATM", "ATR", "DNAPK" or "mTOR" relate to both human and other proteins of this family (Shilo, 2003. Nature Reviews Cancer 3, 155-168). The expression especially includes functionally active derivatives thereof, or functionally active fragments thereof, or a homologues thereof, or variants encoded by a nucleic acid that hybridizes to the nucleic acid encoding said protein under low stringency conditions. Preferably, these low stringency conditions include hybridization in a buffer comprising 35% formamide, 5X SSC, 50 mM Tris-HCl (pH 7.5), 5 mM EDTA, 0.02% PVP, 0.02% BSA, 100 ug/ml denatured salmon sperm DNA, and 10% (wt/vol) dextran sulfate for 18-20 hours at 40°C, washing in a buffer consisting of 2X SSC, 25 mM Tris-HCl (pH 7.4), 5 mM EDTA, and 0.1% SDS for 1-5 hours at 55°C, and washing in a buffer consisting of 2X SSC, 25 mM Tris-HCl (pH 7.4) 5 mM EDTA, and 0.1 % SDS for 1.5 hours at 60°C.

3-(2- {2-[2-(2-amino-ethoxy)-ethoxy]-ethoxy} -ethoxy)-N-[5-(4-chloro-3-methanesulfonyl-phenyl)-4-methyl-thiazol-2yl]-propionamide or a salt thereof is a ligand for all isoforms of PI3K (see above). However, throughout the invention, it is preferred that PI3K is PI3K gamma or PI3K delta, especially the human isoforms thereof.

In some aspects of the invention, first a protein preparation containing PI3K is provided. The methods of the present invention can be performed with any protein preparation as a starting material, as long as the PI3K is solubilized in the preparation. Examples include a liquid mixture of several proteins, a cell lysate, a partial cell lysate which contains not all proteins present in the original cell or a combination of several cell lysates, in particular in cases where not every target protein of interest is present in every cell lysate. The term "protein preparation" also includes dissolved purified protein.

The presence of PI3K protein species in a protein preparation of interest can be detected on Western blots probed with antibodies that are specifically directed against PI3K. In case that PI3K is a specific isoform (e.g. PIK3 gamma and/or PI3K delta), the presence of said isoform can be determined by an isoform-specific antibody. Such antibodies are known in the art (Sasaki et al., 2000, Nature 406, 897-902; Deora et al., 1998, J. Biol. Chem. 273, 29923-29928). Alternatively, also mass spectrometry (MS) could be used (see below).

The presence of ATM, ATR, DNAPK and/or mTOR protein in a protein preparation of interest can be detected on Western blots probed with antibodies that are specific for said protein.

Cell lysates or partial cell lysates can be obtained by isolating cell organelles (e.g. nucleus, mitochondria, ribosomes, golgi etc.) first and then preparing protein preparations derived from these organelles. Methods for the isolation of cell organelles are known in the art (Chapter 4.2 Purification of Organelles from Mammalian Cells in "Current Protocols in Protein Science", Editors: John.E. Coligan, Ben M. Dunn, Hidde L. Ploegh, David W. Speicher, Paul T. Wingfield; Wiley, ISBN: 0-471-14098-8).

In addition, protein preparations can be prepared by fractionation of cell extracts thereby enriching specific types of proteins such as cytoplasmic or membrane proteins (Chapter 4.3 Subcellular Fractionation of Tissue Culture Cells in "Current Protocols in Protein Science", Editors: John.E. Coligan, Ben M. Dunn, Hidde L. Ploegh, David W. Speicher, Paul T. Wingfield; Wiley, ISBN: 0-471-14098-8).

Furthermore protein preparations from body fluids can be used (e.g. blood, cerebrospinal fluid, peritoneal fluid and urine).

For example whole embryo lysates derived from defined development stages or adult stages of model organisms such as C. elegans can be used. In addition, whole organs such as heart dissected from mice can be the source of protein preparations. These organs can also be perfused in vitro in order to obtain a protein preparation.

Furthermore, the protein preparation may be a preparation containing PI3K which has been recombinantely produced. Methods for the production of recombinant proteins in prokaryotic and eukaryotic cells are widely established (Chapter 5 Production of Recombinant Proteins in "Current Protocols in Protein Science", Editors: John. E. Coligan, Ben M. Dunn, Hidde L. Ploegh, David W. Speicher, Paul T. Wingfield; Wiley, 1995, ISBN: 0-471-14098-8).

In a preferred embodiment of the methods of the invention, the provision of a protein preparation includes the steps of harvesting at least one cell containing PI3K and lysing the cell.

Suitable cells for this purpose are e.g. those cells or tissues were members of the PIK3 family are expressed. Members of the PI3K family are expressed in most cells and tissues. PI3K gamma is preferentially expressed in cells of the hematopoietic system (e.g. granulocytes, macrophages, mast cells and platelets) but also in cardiomyocytes, vascular smooth muscle and vascular epithelium cells. PI3K delta is ubiquitously expressed with pronounced expression in lymphocytes, granulocytes and mast cells.

Therefore, in a preferred embodiment, cells isolated from peripheral blood represent a suitable biological material. Procedures for the preparation and culture of human lymphocytes and lymphocyte subpopulations obtained from peripheral blood (PBLs) are widely known (W.E Biddison, Chapter 2.2 "Preparation and culture of human lymphocytes" in Current Protocols in Cell Biology, 1998, John Wiley & Sons, Inc.). For example, density gradient centrifugation is a method for the separation of lymphocytes from other blood cell populations (e.g. erythrocytes and granulocytes). Human lymphocyte subpopulations can be isolated via their specific cell surface receptors which can be recognized by monoclonal antibodies. The physical separation method involves coupling of these antibody reagents to magnetic beads which allow the enrichment of cells that are bound by these antibodies (positive selection). The isolated lymphocyte cells can be further cultured and stimulated by adding antibodies directed against the T-cell receptor or co-receptors such as CD-3 to initiate T-cell recptor signaling and subsequently phosphorylation of PI3K (Houtman et al., 2005, The Journal of Immunology 175(4), 2449-2458).

As an alternative to primary human cells cultured cell lines (e.g. MOLT-4 cells or rat basophilic leukemia (RBL-2H3) cells) can be used. RBL-2H3 cells can be stimulated by cross-linking the high-affinity receptor for IgE (FcepsilonRI) by multivalent antigens to induce activation of PI3K (Kato et al., 2006, J. Immunol. 177(1): 147-154).

In a preferred embodiment, the cell is part of a cell culture system and methods for the harvest of a cell out of a cell culture system are known in the art (literature supra).

The choice of the cell will mainly depend on the expression of PI3K, since it has to be ensured that the protein is principally present in the cell of choice. In order to determine whether a given cell is a suitable starting system for the methods of the invention, methods like Westernblot, PCR-based nucleic acids detection methods, Northemblots and DNA-microarray methods ("DNA chips") might be suitable in order to determine whether a given protein of interest is present in the cell.

The choice of the cell may also be influenced by the purpose of the study. If the in vivo efficacy for a given drug needs to be analyzed then cells or tissues may be selected in which the desired therapeutic effect occurs (e.g. granulocytes or mast cells). By contrast, for the elucidation of protein targets mediating unwanted side effects the cell or tissue may be analysed in which the side effect is observed (e.g. cardiomycytes, vascular smooth muscle or epithelium cells).

Furthermore, it is envisaged within the present invention that the cell containing PI3K may be obtained from an organism, e.g. by biopsy. Corresponding methods are known in the art. For example, a biopsy is a diagnostic procedure used to obtain a small amount of tissue, which can then be examined miscroscopically or with biochemical methods. Biopsies are important to diagnose, classify and stage a disease, but also to evaluate and monitor drug treatment.

It is encompassed within the present invention that by the harvest of the at least one cell, the lysis is performed simultaneously. However, it is equally preferred that the cell is first harvested and then separately lysed.

Methods for the lysis of cells are known in the art (Karwa and Mitra: Sample preparation for the extraction, isolation, and purification of Nuclei Acids; chapter 8 in "Sample Preparation Techniques in Analytical Chemistry", Wiley 2003, Editor: Somenath Mitra, print ISBN: 0471328456; online ISBN: 0471457817). Lysis of different cell types and tissues can be achieved by homogenizers (e.g. Potter-homogenizer), ultrasonic desintegrators, enzymatic lysis, detergents (e.g. NP-40, Triton X-100, CHAPS, SDS), osmotic shock, repeated freezing and thawing, or a combination of these methods.

According to the methods of the invention, the protein preparation containing PI3K is contacted with the 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide immobilized on a solid support under conditions allowing the formation of a 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide - PI3K complex.

In the present invention, the term "3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide - PI3K complex" denotes a complex where a 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide interacts with PI3K, e.g. by covalent or, most preferred, by non-covalent binding. The same definition applies also for complexes between a 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide and ATM, ATR, DNAPK or mTOR.

The skilled person will know which conditions can be applied in order to enable the formation of the 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide- PI3K complex.

In the context of the present invention, the term "under conditions allowing the formation of the complex" includes all conditions under which such formation, preferably such binding is possible. This includes the possibility of having the solid support on an immobilized phase and pouring the lysate onto it. In another preferred embodiment, it is also included that the solid support is in a particulate form and mixed with the cell lysate.

In the context of non-covalent binding, the binding between 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide and PI3K is, e.g., via salt bridges, hydrogen bonds, hydrophobic interactions or a combination thereof.

In a preferred embodiment, the steps of the formation of the 3-(2-{2-[2-(2-amino-ethoxy)-ethoxy]-ethoxy} -ethoxy)-N-[5-(4-chloro-3-methanesulfonyl-phenyl)-4-methyl-thiazol-2yl]-propionamide - PI3K complex are performed under essentially physiological conditions. The physical state of proteins within cells is described in Petty, 1998 (Howard R. Petty, Chapter 1, Unit 1.5 in: Juan S. Bonifacino, Mary Dasso, Joe B. Harford, Jennifer Lippincott-Schwartz, and Kenneth M. Yamada (eds.) Current Protocols in Cell Biology Copyright © 2003 John Wiley & Sons, Inc. All rights reserved. DOI: 10.1002/0471143030.cb0101s00Online Posting Date: May, 2001Print Publication Date: October, 1998).

The contacting under essentially physiological conditions has the advantage that the interactions between the ligand, the cell preparation (i. e. the kinase to be characterized) and optionally the compound reflect as much as possible the natural conditions. "Essentially physiological conditions" are *inter alia* those conditions which are present in the original, unprocessed sample material. They include the physiological protein concentration, pH, salt concentration, buffer capacity and post-translational modifications of the proteins involved. The term "essentially physiological conditions" does not require conditions identical to those in the original living organism, wherefrom the sample is derived, but essentially cell-like conditions or conditions close to cellular conditions. The person skilled in the art will, of course, realize that certain constraints may arise due to the experimental set-up which will eventually lead to less cell-like conditions. For example, the eventually necessary disruption of cell walls or cell membranes when taking and processing a sample from a living organism may require conditions which are not identical to the physiological conditions found in the organism. Suitable variations of physiological conditions for practicing the methods of the invention will be apparent to those skilled in the art and are encompassed by the term "essentially physiological conditions" as used herein. In summary, it is to be understood that the term "essentially physiological conditions" relates to conditions close to physiological conditions, as e. g. found in natural cells, but does not necessarily require that these conditions are identical.

For example, "essentially physiological conditions" may comprise 50-200 mM NaCl or KCl, pH 6.5-8.5, 20-37°C, and 0.001-10 mM divalent cation (e.g. Mg++, Ca++,); more preferably about 150 m NaCl or KCl, pH7.2 to 7.6, 5 mM divalent cation and often include 0.01-1.0 percent non-specific protein (e.g. BSA). A non-ionic detergent (Tween, NP-40, Triton-X100) can often be present, usually at about 0.001 to 2%, typically 0.05-0.2% (volume/volume). For general guidance, the following buffered aequous conditions may be applicable: 10-250 mM NaCl, 5-50 mM Tris HCl, pH5-8, with optional addition of divalent cation(s) and/or metal chelators and/or non-ionic detergents.

Preferably, "essentially physiological conditions" mean a pH of from 6.5 to 7.5, preferably from 7.0 to 7.5, and / or a buffer concentration of from 10 to 50 mM, preferably from 25 to 50 mM, and / or a concentration of monovalent salts (e.g. Na or K) of from 120 to 170 mM, preferably 150 mM, Divalent salts (e.g. Mg or Ca) may further be present at a concentration of from 1 to 5 mM, preferably 1 to 2 mM, wherein more preferably the buffer is selected from the group consisting of Tris-HCl or HEPES.

In the context of the present invention, 3-(2-{2-[2-(2-amino-ethoxy)-ethoxy]-ethoxy}-ethoxy)-N-[5-(4-chloro-3-methanesulfonyl-phenyl)4-methyl-thiazol-2yl]-propionamide or a salt thereof is immobilized on a solid support. Throughout the invention, the term "solid support" relates to every undissolved support being able to immobilize a small molecule ligand on its surface.

According to a further preferred embodiment, the solid support is selected from the group consisting of agarose, modified agarose, sepharose beads (e.g. NHS-activated sepharose), latex, cellulose, and ferro- or ferrimagnetic particles.

3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide or a salt thereof may be coupled to the solid support either covalently or non-covalently. Non-covalent binding includes binding via biotin affinity ligands binding to steptavidin matrices.

Preferably, the 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide or salt thereof is covalently coupled to the solid support.

Before the coupling, the matrixes can contain active groups such as NHS, Carbodimide etc. to enable the coupling reaction with the phenylthiazole ligand 1. The 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide or a salt thereof can be coupled to the solid support by direct coupling (e.g. using functional groups such as amino-, sulfhydryl-, carboxyl-, hydroxyl-, aldehyde-, and ketone groups) and by indirect coupling (e.g. via biotin, biotin being covalently attached to 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide or a salt thereof and non-covalent binding of biotin to streptavidin which is bound to solid support directly).

The linkage to the solid support material may involve cleavable and non-cleavable linkers. The cleavage may be achieved by enzymatic cleavage or treatment with suitable chemical methods.

Preferred binding interfaces for binding 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide or a salt thereof to solid support material are linkers with a C-atom backbone. Typically linkers have backbone of 8, 9 or 10 atoms. The linkers contain either a carboxy- or amino-active group.

The skilled person will appreciate that between the individual steps of the methods of the invention, washing steps may be necessary. Such washing is part of the knowledge of the person skilled in the art. The washing serves to remove non-bound components of the cell lysate from the solid support. Nonspecific (e.g. simple ionic) binding interactions can be minimized by adding low levels of detergent or by moderate adjustments to salt concentrations in the wash buffer.

According to the identification methods of the invention, the read-out system is either the detection or determination of PI3K (first aspect of the invention), the detection of the 3-(2-{2-[2-(2-amino-ethoxy)-ethoxy]-ethoxy}-ethoxy)-N-[5-(4-chloro-3-methanesulfonylphenyl)-4-methyl-thiazol-2yl]-propionamide- PI3K complex (second aspect of the invention), or the determination of the amount of the 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide - PI3K complex (second, third and forth aspect of the invention).

Throughout the invention, the same read-out systems used for the determination or detection of PI3K, the detection of the 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide-PI3K complex or the determination of the amount of the 3-(2-{2-[2-(2-amino-ethoxy)-ethoxy]-ethoxy} -ethoxy)-N-[5-(4-chloro-3-methanesulfonyl-phenyl)-4-methyl-thiazol-2yl]-propionamide - PI3K complex can be used for the detection of ATM, ATR, DNAPK or mTOR or the detection or the determination of the amount of a complex between 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide and said proteins. This implies that in cases where an agent specific for PI3K (e.g. an antibody) is used, an agent specific for ATM, ATR, DNAPK, or mTOR has to be used instead. Consequently, the embodiments and explanations given below also apply to the detection of ATM, ATR, DNAPK or mTOR or to the detection of the complex or to the determination of the amount of a complex between 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide and said proteins.

In the method according to the first aspect of the invention, the detection or determination of separated PI3K is preferably indicative for the fact that the compound is able to separate PI3K from the immobilized 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide or a salt thereof. This capacity indicates that the respective compound interacts, preferably binds to PI3K, which is indicative for its therapeutic potential.

In one embodiment of the method according to the second aspect, the 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide -PI3K complex formed during the method of the invention is detected. The fact that such complex is formed preferably indicates that the compound does not completely inhibit the formation of the complex. On the other hand, if no complex is formed, the compound is presumably a strong interactor with PI3K, which is indicative for its therapeutic potential.

According to the methods of the second, third and forth aspect of the invention the amount of the 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide-PI3K complex formed during the method is determined. In general, the less complex in the presence of the respective compound is formed, the stronger the respective compound interacts with PI3K, which is indicative for its therapeutic potential.

The detection of the 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide - PI3K complex according to the second aspect of the invention can be performed by using labeled antibodies directed against PI3K and a suitable readout system.

According to a preferred embodiment of the second aspect of the invention, the 3-(2-{2-[2-(2-amino-ethoxy)-ethoxy]-ethoxy}-ethoxy)-N-[5-(4-chloro-3-methanesulfonylphenyl)-4-methyl-thiazol-2yl]-propionamide - PI3K complex complex is detected by determining its amount.

In the course of the second, third and forth aspect of the invention, it is preferred that PI3K is separated from the immobilized 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide in order to determine the amount of the 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide - PI3K complex.

According to invention, separating means every action which destroys the interactions between 3-(2-{2-[2-(2-amino-ethoxy)-ethoxy]-ethoxy} -ethoxy)-N-[5-(4-chloro-3-methanesulfonyl-phenyl)-4-methyl-thiazol-2yl]-propionamide and PI3K. This includes in a preferred embodiment the elution of PI3K from the immobilized 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide.

The elution can be achieved by using non-specific reagents as described in detail below (ionic strength, pH value, detergents). In addition, it can be tested whether a compound of interest can specifically elute the PI3K from 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide. Such PI3K interacting compounds are described further in the following sections.

Such non-specific methods for destroying the interaction are principally known in the art and depend on the nature of the ligand enzyme interaction. Principally, change of ionic strength, the pH value, the temperature or incubation with detergents are suitable methods to dissociate the target enzymes from the immobilized ligand. The application of an elution buffer can dissociate binding partners by extremes of pH value (high or low pH; e.g. lowering pH by using 0.1 M citrate, pH2-3), change of ionic strength (e.g. high salt concentration using NaI, KI, MgCl2, or KCI), polarity reducing agents which disrupt hydrophobic interactions (e.g. dioxane or ethylene glycol), or denaturing agents (chaotropic salts or detergents such as Sodium-docedyl-sulfate, SDS; Review: Subramanian A., 2002, Immunoaffinty chromatography).

In some cases, the solid support has preferably to be separated from the released material. The individual methods for this depend on the nature of the solid support and are known in the art. If the support material is contained within a column the released material can be collected as column flowthrough. In case the support material is mixed with the lysate components (so called batch procedure) an additional separation step such as gentle centrifugation may be necessary and the released material is collected as supernatant. Alternatively magnetic beads can be used as solid support so that the beads can be eliminated from the sample by using a magnetic device.

In step d) of the method according to the first aspect of the invention, it is determined if PI3K has been separated from the immobilized 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide. This may include the detection of PI3K or the determination of the amount PI3K.

Consequently, at least in preferred embodiments of all identification methods of the invention, methods for the detection of separated PI3K or for the determination of its amount are used. Such methods are known in the art and include physico-chemical methods such as protein sequencing (e.g. Edmann degradation), analysis by mass spectrometry methods or immunodetection methods employing antibodies directed against PI3K.

Throughout the invention, if an antibody is used in order to detect PI3K or in order to determine its amount (e.g. via ELISA), the skilled person will understand that, if a specific isoform of PI3K is to be detected or if the amount of a specific isoform of PI3K is to be determined, an isoform-specific antibody may be used. As indicated above, such antibodies are known in the art. Furthermore, the skilled person is aware of methods for producing the same.

Preferably, PI3K, ATM, ATR, DNAPK and/or mTOR are detected or the amount of said proteins is determined by mass spectrometry or immunodetection methods. In the following, this will be explained in more detail by reference to PI3K, but the embodiments and explanation described below also apply to ATM, ATR, DNAPK or mTOR.

The identification of proteins with mass spectrometric analysis (mass spectrometry) is known in the art (Shevchenko et al., 1996, Analytical Chemistry 68: 850-858; Mann et al., 2001, Analysis of proteins and proteomes by mass spectrometry, Annual Review of Biochemistry 70, 437-473) and is further illustrated in the example section.

Preferably, the mass spectrometry analysis is performed in a quantitative manner, for example by using iTRAQ technology (isobaric tags for relative and absolute quatification) or cICAT (cleavable isotope-coded affinity tags) (Wu et al., 2006. J. Proteome Res. 5, 651-658).

According to a further preferred embodiment of the present invention, the characterization by mass spectrometry (MS) is performed by the identification of proteotypic peptides of PI3K. The idea is that PI3K is digested with proteases and the resulting peptides are determined by MS. As a result, peptide frequencies for peptides from the same source protein differ by a great degree, the most frequently observed peptides that "typically" contribute to the identification of this protein being termed "proteotypic peptide". Therefore, a proteotypic peptide as used in the present invention is an experimentally well observable peptide that uniquely identifies a specific protein or protein isoform.

According to a preferred embodiment, the characterization is performed by comparing the proteotypic peptides obtained in the course of practicing the methods of the invention with known proteotypic peptides. Since, when using fragments prepared by protease digestion for the identification of a protein in MS, usually the same proteotypic peptides are observed for a given enzyme, it is possible to compare the proteotypic peptides obtained for a given sample with the proteotypic peptides already known for enzymes of a given class of enzymes and thereby identifying the enzyme being present in the sample.

As an alternative to mass spectrometry analysis, the eluted PI3K (including coeluted binding partners or scaffold proteins), can be detected or its amount can be determined by using a specific antibody directed against PI3K (or against an isoform of PI3K, see above).

Furthermore, in another preferred embodiment, once the identity of the coeluted binding partner has been established by mass spectrometry analysis, each binding partner can be detected with specific antibodies directed against this protein.

Suitable antibody-based assays include but are not limited to Western blots, ELISA assays, sandwich ELISA assays and antibody arrays or a combination thereof. The establishment of such assays is known in the art (Chapter 11, Immunology, pages 11-1 to 11-30 in: Short Protocols in Molecular Biology. Fourth Edition, Edited by F.M. Ausubel et al., Wiley, New York, 1999).

These assays can not only be configured in a way to detect and quantify a PI3K interacting protein of interest (e.g. a catalytic or regulatory subunit of a PI3K complex), but also to analyse posttranslational modification patterns such as phosphorylation or ubiquitin modification.

Furthermore, the identification methods of the invention involve the use of compounds which are tested for their ability to be an PI3K interacting compound.

Principally, according to the present invention, such a compound can be every molecule which is able to interact with PI3K, eg. by inhibiting its binding to 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide or a salt thereof. Preferably, the compound has an effect on PI3K, e.g. a stimulatory or inhibitory effect.

Preferably, said compound is selected from the group consisting of synthetic or naturally occurring chemical compounds or organic synthetic drugs, more preferably small molecules, organic drugs or natural small molecule compounds. Preferably, said compound is identified starting from a library containing such compounds. Then, in the course of the present invention, such a library is screened.

Such small molecules are preferably not proteins or nucleic acids. Preferably, small molecules exhibit a molecular weight of less than 1000 Da, more preferred less than 750 Da, most preferred less than 500 Da.

A "library" according to the present invention relates to a (mostly large) collection of (numerous) different chemical entities that are provided in a sorted manner that enables both a fast functional analysis (screening) of the different individual entities, and at the same time provide for a rapid identification of the individual entities that form the library. Examples are collections of tubes or wells or spots on surfaces that contain chemical compounds that can be added into reactions with one or more defined potentially interacting partners in a high-throughput fashion. After the identification of a desired "positive" interaction of both partners, the respective compound can be rapidly identified due to the library construction. Libraries of synthetic and natural origins can either be purchased or designed by the skilled artisan.

Examples of the construction of libraries are provided in, for example, Breinbauer R, Manger M, Scheck M, Waldmann H. Natural product guided compound library development. Curr Med Chem. 2002 Dec;9(23):2129-45, wherein natural products are described that are biologically validated starting points for the design of combinatorial libraries, as they have a proven record of biological relevance. This special role of natural products in medicinal chemistry and chemical biology can be interpreted in the light of new insights about the domain architecture of proteins gained by structural biology and bioinformatics. In order to fulfill the specific requirements of the individual binding pocket within a domain family it may be necessary to optimise the natural product structure by chemical variation. Solid-phase chemistry is said to become an efficient tool for this optimisation process, and recent advances in this field are highlighted in this review article. Other related references include Edwards PJ, Morrell AI. Solid-phase compound library synthesis in drug design and development. Curr Opin Drug Discov Devel. 2002 Jul;5(4):594-605.; Merlot C, Domine D, Church DJ. Fragment analysis in small molecule discovery. Curr Opin Drug Discov Devel. 2002 May;5(3):391-9. Review; Goodnow RA Jr. Current practices in generation of small molecule new leads. J Cell Biochem Suppl. 2001;Suppl 37:13-21; which describes that the current drug discovery processes in many pharmaceutical companies require large and growing collections of high quality lead structures for use in high throughput screening assays. Collections of small molecules with diverse structures and "drug-like" properties have, in the past, been acquired by several means: by archive of previous internal lead optimisation efforts, by purchase from compound vendors, and by union of separate collections following company mergers. Although high throughput/combinatorial chemistry is described as being an important component in the process of new lead generation, the selection of library designs for synthesis and the subsequent design of library members has evolved to a new level of challenge and importance. The potential benefits of screening multiple small molecule compound library designs against multiple biological targets offers substantial opportunity to discover new lead structures.

In a preferred embodiment of the second and third aspect, the PI3K containing protein preparation is first incubated with the compound and then with the immobilized 3-(2-{2-[2-(2-amino-ethoxy)-ethoxy]-ethoxy}-ethoxy)-N-[5-(4-chloro-3-methanesulfonylphenyl)-4-methyl-thiazol-2yl]-propionamide. However, the simultaneous incubation of the compound and the immobilized 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide (coincubation) with the PI3K containing protein preparation is equally preferred (competitive binding assay).

In case that the incubation with the compound is first, the PI3K is preferably first incubated with the compound for 10 to 60 minutes, more preferred 30 to 45 minutes at a temperature of 4°C to 37°C, more preferred 4°C to 25°C, most preferred 4°C. Preferably compounds are used at concentrations ranging from 1 µM to 1 mM, preferably from 10 to 100 µM. The second step, contacting with the immobilized ligand, is preferably performed for 10 to 60 minutes at 4°C.

In case of simultaneous incubation, the PI3K is preferably simultaneously incubated with the compound and 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide for 30 to 120 minutes, more preferred 60 to 120 minutes at a temperature of 4°C to 37°C, more preferred 4°C to 25°C, most preferred 4°C. Preferably compounds are used at concentrations ranging from 1 µM to I mM, preferably from 10 to 100 µM.

Furthermore, steps a) to c) of the second aspect of the invention may be performed with several protein preparations in order to test different compounds. This embodiment is especially interesting in the context of medium or high troughput screenings (see below).

In a preferred embodiment of the method of the invention according to the third or forth aspect, the amount of the 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide - PI3K complex formed in step c) is compared to the amount formed in step b)

In a preferred embodiment of the method of the invention according to the third or forth aspect, a reduced amount of the a 3-(2-{2-[2-(2-amino-ethoxy)-ethoxy]-ethoxy}-ethoxy)-N-[3-(4-chloro-3-methanesulfonyl-phenyl)-4-methyl-thiazol-2yl]-propionamide- PI3K complex formed in step c) in comparison to step b) indicates that PI3K is a target of the compound. This results from the fact that in step c) of this method of the invention, the compound competes with the ligand for the binding of PI3K. If less PI3K is present in the aliquot incubated with the compound, this means preferably that the compound has competed with the inhibitor for the interaction with the enzyme and is, therefore, a direct target of the protein and vice versa.

Preferably, the identification methods of the invention are performed as a medium or high throughput screening.

The interaction compound identified according to the present invention may be further characterized by determining whether it has an effect on PI3K, for example on its kinase activity (Carpenter et al., 1990, J. Biol. Chem. 265, 19704-19711). Such assays are known in the art, also in a format that allows medium to high throughput screening (Fuchikami et al., 2002, J. Biomol. Screening 7, 441-450).

In addition, the interaction compound identified according to the present invention may be further characterized by determining whether it has an effect on ATM, ATR, DNAPK or mTOR for example on their kinase activities (Knight et al., 2004. Bioorganic and Medicinal Chemistry 12, 4749-4759; Knight et al., 2006, Cell 125, 733-747).

Briefly, PI3K lipid kinase activity can be measured using solution-based assays with phopholipid vesicles. The reaction is terminated by the addition of acidified organic solvents and subsequent phase separation by extraction or thin layer chromatography analysis (Carpenter et al., 1990, J. Biol. Chem. 265, 19704-19711).

Alternatively, a fluorescence polarization assay format can be used. Briefly, PI3K is incubated with a suitable phosphoinositol substrate. After the reaction is complete the reaction products are mixed with a specfic phosphoinositol detector protein and a fluorescent phosphoinositol probe. The polarization (mP) values decrease as probe binding to the phosphoinositol detector protein is displaced by the reaction product. The degree of polarization of the fluorescent probe is inversely proportional to the amount of the product of the PI3K reaction (Drees et al., 2003, Comb. Chem. High Throughput Screening 6, 321-330).

For the determination of PI3K protein kinase activity a fluorescence polarization assay with a suitable peptide substratecan be used. Briefly, a fluorescein-labeled peptide substrate may be incubated with PI3K (e.g. PI3K delta), ATP and an anti-phosphoserine antibody. As the reaction proceeds, the phosphorylated peptide binds to the anti-phosphoserine antibody, resulting in an increase in the polarization signal. Compounds that inhibit the kinase result in a low polarization signal.

The compounds identified according to the present invention may further be optimized (lead optimisation). This subsequent optimisation of such compounds is often accelerated because of the structure-activity relationship (SAR) information encoded in these lead generation libraries. Lead optimisation is often facilitated due to the ready applicability of high-throughput chemistry (HTC) methods for follow-up synthesis.

One example of such a library and lead optimization is described for PI3K gamma (Pomel et al., 2006, J. Med. Chem. 49, 3857-3871).

The methods of the invention comprise a method step wherein it is determined whether the compound is able to separate also ATM, ATR, DNAPK and/or mTOR from the immobilized 3-(2- {2-[2-(2-amino-ethoxy)-ethoxy]-ethoxy} -ethoxy)-N-[5-(4-chloro-3-methanesulfonyl-phenyl)-4-methyl-thiazol-2yl]-propionamide (first aspect) or whether also a complex between 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide and ATM, ATR, DNAPK and/or mTOR has been formed. As indicated above, these steps can essentially be performed as described above for PI3K, where agents, e.g. antibodies specific for the given kinase are used when required.

The rational behind these method steps is that it is possible to determine the specificity of the identified PI3K interacting compound. It is preferred, in the context of the present invention, to identify PI3K interacting compounds which are specific for PI3K, i.e. which bind to a lesser extend to ATM, ATR, DNAPK and/or mTOR, or, even more preferred, do not bind to one of or all of these proteins.

The invention further discloses a method for the preparation of a pharmaceutical composition comprising the steps of
a) identifying a PI3K interacting compound as described above, and
b) formulating the interacting compound to a pharmaceutical composition.

Therefore, the invention provides a method for the preparation of pharmaceutical compositions, which may be administered to a subject in an effective amount. In a preferred aspect, the therapeutic is substantially purified. The subject to be treated is preferably an animal including, but not limited to animals such as cows, pigs, horses, chickens, cats, dogs, etc., and is preferably a mammal, and most preferably human. In a specific embodiment, a non-human mammal is the subject.

The compounds identified according to the invention are useful for the prevention or treatment of diseases where PI3K plays a role such as cancer (e.g. breast, colon or ovary cancer), metabolic disorders (e.g. diabetes or obesity) or autoimmune/inflammatory disorders (e.g. rheumatic arthritis, psoriasis, Crohn's disease, ulcerative colitis, asthma or allergic reactions).

Consequently, the present invention also relates to the use of a compound identified by the methods of the invention for the preparation of a medicament for the treatment of one or more of the above mentioned diseases. Furthermore, the present invention relates to a pharmaceutical composition comprising said compound.

In general, the pharmaceutical compositions of the present invention comprise a therapeutically effective amount of a therapeutic, and a pharmaceutically acceptable carrier. In a specific embodiment, the term "pharmaceutically acceptable" means approved by a regulatory agency of the Federal or a state government or listed in the U.S. Pharmacopeia or other generally recognized pharmacopeia for use in animals, and more particularly, in humans. The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, including but not limited to peanut oil, soybean oil, mineral oil, sesame oil and the like. Water is a preferred carrier when the pharmaceutical composition is administered orally. Saline and aqueous dextrose are preferred carriers when the pharmaceutical composition is administered intravenously. Saline solutions and aqueous dextrose and glycerol solutions are preferably employed as liquid carriers for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsions, tablets, pills, capsules, powders, sustained-release formulations and the like The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" by E.W. Martin. Such compositions will contain a therapeutically effective amount of the therapeutic, preferably in purified form, together with a suitable amount of carrier so as to provide the form for proper administration to the patient. The formulation should suit the mode of administration.

In a preferred embodiment, the composition is formulated, in accordance with routine procedures, as a pharmaceutical composition adapted for intravenous administration to human beings. Typically, compositions for intravenous administration are solutions in sterile isotonic aqueous buffer. Where necessary, the composition may also include a solubilizing agent and a local anesthetic such as lidocaine to ease pain at the site of the injection. Generally, the ingredients are supplied either separately or mixed together in unit dosage form, for example, as a dry lyophilized powder or water-free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water or saline for injection can be provided so that the ingredients may be mixed prior to administration.

The therapeutics of the invention can be formulated as neutral or salt forms. Pharmaceutically acceptable salts include those formed with free carboxyl groups such as those derived from hydrochloric, phosphoric, acetic, oxalic, tartaric acids, etc., those formed with free amine groups such as those derived from isopropylamine, triethylamine, 2-ethylamino ethanol, histidine, procaine, etc., and those derived from sodium, potassium, ammonium, calcium, and ferric hydroxides, etc..

The amount of the therapeutic of the invention which will be effective in the treatment of a particular disorder or condition will depend on the nature of the disorder or condition, and can be determined by standard clinical techniques. In addition, in vitro assays may optionally be employed to help identify optimal dosage ranges. The precise dose to be employed in the formulation will also depend on the route of administration, and the seriousness of the disease or disorder and should be decided according to the judgment of the practitioner and each patient's circumstances. However, suitable dosage ranges for intravenous administration are generally about 20-500 micrograms of active compound per kilogram body weight. Suitable dosage ranges for intranasal administration are generally about 0.01 pg/kg body weight to 1 mg/kg body weight. Effective doses may be extrapolated from dose-response curves derived from in vitro or animal model test systems. In general, suppositories may contain active ingredient in the range of 0.5% to 10% by weight; oral formulations preferably contain 10% to 95% active ingredient.

Various delivery systems are known and can be used to administer a therapeutic of the invention, e.g., encapsulation in liposomes, microparticles, and microcapsules: use of recombinant cells capable of expressing the therapeutic, use of receptor-mediated endocytosis (e.g., Wu and Wu, 1987, J. Biol. Chem. 262:4429-4432); construction of a therapeutic nucleic acid as part of a retroviral or other vector, etc. Methods of introduction include but are not limited to intradermal, intramuscular, intraperitoneal, intravenous, subcutaneous, intranasal, epidural, and oral routes. The compounds may be administered by any convenient route, for example by infusion, by bolus injection, by absorption through epithelial or mucocutaneous linings (e.g., oral, rectal and intestinal mucosa, etc.), and may be administered together with other biologically active agents. Administration can be systemic or local. In addition, it may be desirable to introduce the pharmaceutical compositions of the invention into the central nervous system by any suitable route, including intraventricular and intrathecal injection; intraventricular injection may be facilitated by an intraventricular catheter, for example, attached to a reservoir, such as an Ommaya reservoir. Pulmonary administration can also be employed, e.g., by use of an inhaler or nebulizer, and formulation with an aerosolizing agent.

In a specific embodiment, it may be desirable to administer the pharmaceutical compositions of the invention locally to the area in need of treatment. This may be achieved by, for example, and not by way of limitation, local infusion during surgery, topical application, e.g., in conjunction with a wound dressing after surgery, by injection, by means of a catheter, by means of a suppository, or by means of an implant, said implant being of a porous, non-porous, or gelatinous material, including membranes, such as sialastic membranes, or fibers. In one embodiment, administration can be by direct injection at the site (or former site) of a malignant tumor or neoplastic or preneoplastic tissue.

In another embodiment, the therapeutic can be delivered in a vesicle, in particular a liposome (Langer, 1990, Science 249:1527-1533).

In yet another embodiment, the therapeutic can be delivered via a controlled release system. In one embodiment, a pump may be used (Langer, supra). In yet another embodiment, a controlled release system can be placed in proximity of the therapeutic target, i.e., the brain, thus requiring only a fraction of the systemic dose

In the context of the present invention, it has been found that phenylthiazole ligand 1 is a ligand for ATM, ATR, DNAPK, and mTOR. Therefore, the present invention also relates to methods for the identification of compounds interacting with ATM, ATR, DNAPK and/or mTOR. These methods are performed as described above in the context of the identification of PI3K interacting coumpounds. Furthermore, it is envisaged within the present invention that these methods for the identification of ATM, ATR, DNAPK or mTOR-interacting compounds may or may not contain the step of determining whether a given compound may be able to interact also with other PIKK kinases as defined in the present invention

The invention further relates to a method for the purification of ATM, ATR, DNAPK and/or mTOR, comprising the steps of
a) providing a protein preparation containing one or more of said proteins,
b) contacting the protein preparation with 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide or a salt thereof immobilized on a solid support under conditions allowing the formation of an 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide - protein complex, and
c) separating the protein from the immobilized 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide.

As mentioned above, it has been surprisingly found that phenylthiazole ligand 1 is a ligand which recognizes these proteins. This enables efficient purification methods for these proteins.

The embodiments as defined above for the identification methods of the invention also apply to the purification method of the invention.

Preferably, said purification is performed using an isoform specific antibody as explained above.

In a preferred embodiment, the purification method of the invention further comprises after step c) the identification of proteins being capable of binding to ATM, ATR, DNAPK and/or mTOR. This is especially interesting when the formation of the complex is performed under essentially physiological conditions, because it is then possible to preserve the natural condition of the enzyme which includes the existence of binding partners, enzyme subunits or post-translational modifications, which can then be identified with the help of mass spectrometry (MS).

Consequently, in a preferred embodiment, the purification method of the invention further comprises after step c) the determination whether the given protein is further posttranslationally modified, e.g. by ubiquitin modification.

The invention further relates to the use of 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide or a salt thereof for the identification of ATM, ATR, DNAPK and/or mTOR interacting compounds and for the purification of PI3K. The embodiments as defined above also apply to the uses of the invention.

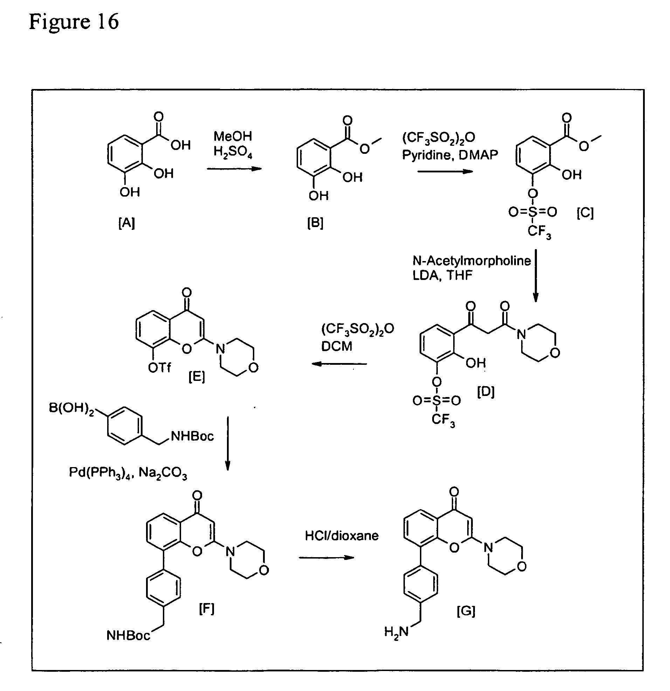

It is also disclosed that not only 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide or a salt thereof, but in addition also another ligand, namely the phenylmorpholin-chromen ligand (8-(4-aminomethyl-phenyl)-2-morpholin-4-yl-chromen-4-one) as shown in Fig. 16, or derivatives thereof, e.g. compounds comprising the identical core but which have another linker, preferably coupled to the nitrogen not being part of the cyclic structures, for linkage to the solid support, may be used for the identification of the interacting compounds. Consequently, both ligands are immobilized. In this context, it is included that, in case that beads are used, both ligands are immobilized on the same bead or that one ligand is immobilized on one bead and the other ligand is immobilized on the other bead. In this context, typically linkers have backbone of 8, 9 or 10 atoms. The linkers may contain either a carboxy-, hydroxy or amino-active group.

The invention is further illustrated by the following figures and examples, which are not considered as being limiting for the scope of protection conferred by the claims of the present application.

### Short description of the figures

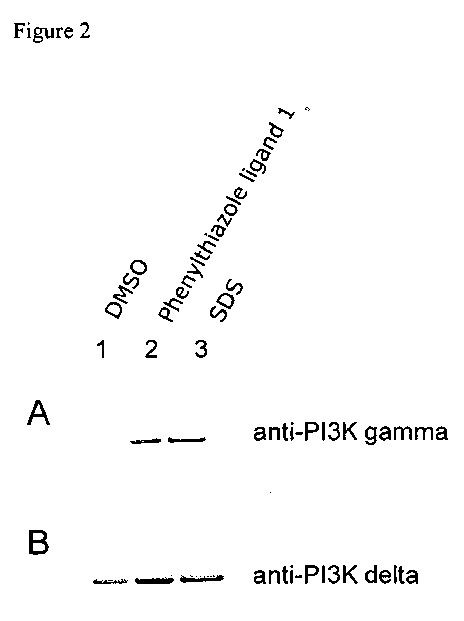

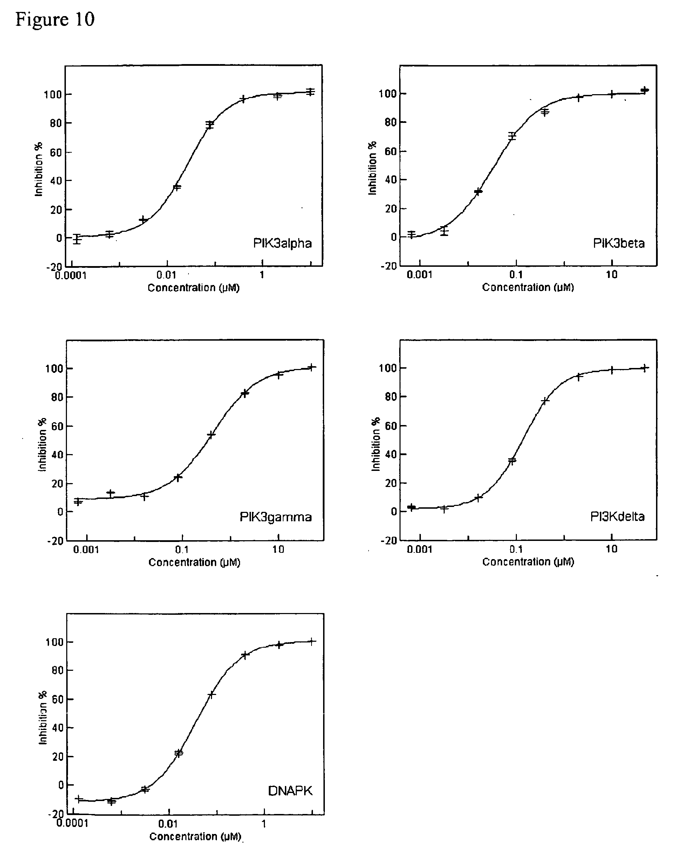

**Figure 1****:** Synthesis and structure of phenylthiazole ligand 1.
   The phenylthiazole ligand 1 was synthesized as described in example 1.
**Figure 2****:** Drug pulldown experiment with immobilized phenylthiazole ligand 1 and Western blot detection of PI3K proteins.
   As biological material a cell lysate prepared from MOLT-4 cells was used. The drug pulldown experiment was performed as described in Example 2 with lysate samples containing 50 mg of protein. Captured proteins were eluted with DMSO containing buffer (lane 1), 100 µM of free phenylthiazole ligand 1 or SDS sample buffer (lane 3). The eluted samples were separated on SDS-polyacrylamide gels and transferred to membranes. The blots were first incubated with specific antibodies directed against PI3K gamma (Figure 2A) and PI3K delta (Figure 2B). Secondary detection antibodies labeled with fluorescent dyes for detection were used with the Odyssey infrared imaging system. Lane 1: DMSO elution control; lane 2: elution with 100 µM free phenylthiazole ligand 1; lane 3: SDS elution.

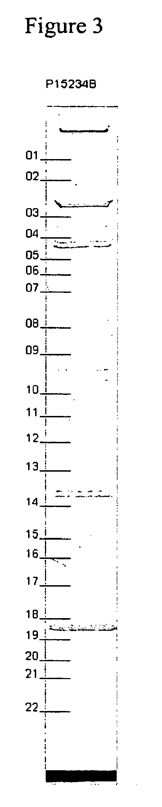

**Figure 3****:** Drug pulldown experiment with immobilized phenylthiazole ligand 1 for mass spectrometry analysis of proteins.
   A protein gel after staining with Coomassie blue is shown. The indicated gel areas were cut out as gel slices and proteins were subjected to analysis by mass spectrometry.
   The drug pulldown experiment was performed as described in Example 2 with a MOLT-4 cell lysate sample containing 50 mg of protein. Proteins bound to immobilized phenylthiazole ligand 1 were eluted with SDS sample buffer and separated by SDS-polyacrylamide gel electrophoresis (SDS-PAGE).
**Figure 4****:** Peptides identified of PI3K gamma.
   The peptides that were identified by mass spectrometry analysis of the human PI3K delta sequence are shown in bold type and underlined.
**Figure 5****:** Peptides identified of PI3K delta.
   The peptides that were identified by mass spectrometry analysis of the human PI3K gamma sequence are shown in bold type and underlined.

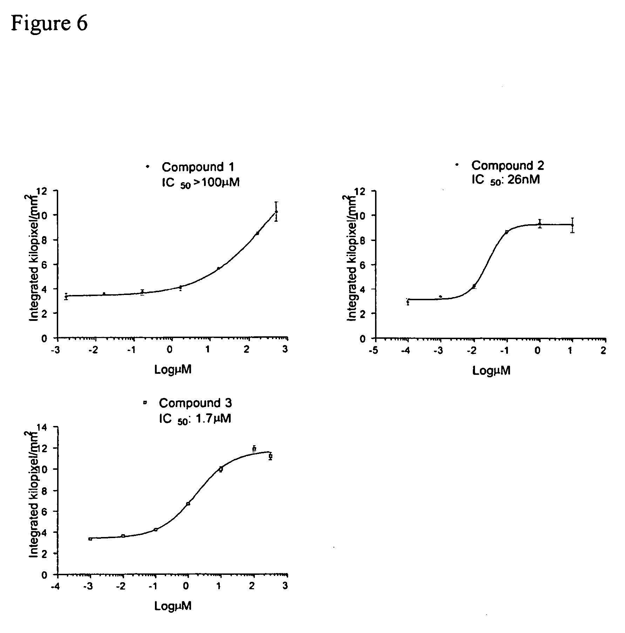

**Figure 6****:** Elution assay for the identification of PI3K gamma interacting compounds.
   The experiment was performed as described in example 3. PI3K gamma protein was captured by immobilized phenylthiazole ligand 1 from MOLT-4 cell lysate and eluted by the compounds as indicated. Eluates were transferred to a nitrocellulose membrane and PI3K gamma was detected with the Odyssey Infrared Imaging system. First antibody: anti-PI3K gamma (Jena Bioscience ABD-026S; mouse antibody). Second antibody: anti-mouse IRDye800 (Rockland, 610-732-124). Integrated Intensity (integrated kilopixel/mm²) are shown.
   Compounds used for elution:
   Compound 1 (LY294002); IC₅₀ > 100 µM; compound 2 (AS-605240): IC₅₀=26 nM; compound 3 (AS-604850); IC₅₀=1.7 µM,

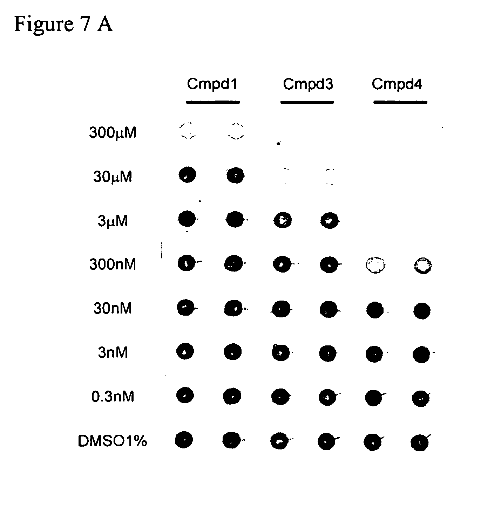

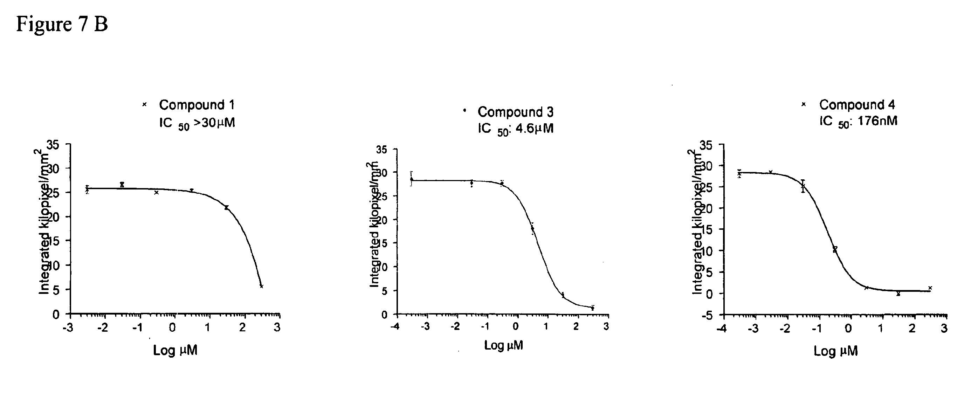

**Figure 7****:** Competitive binding assay for the identification of PI3K gamma interacting compounds.
   The experiment was performed as described in example 4. Test compounds at the indicated concentrations and the affinity matrix were added to MOLT-4 cell lysate and the PI3K gamma protein not interacting with test compounds was captured by the immobilized phenylthiazole ligand 1 on the affinity matrix. The affinity matrix was separated from the lysate, bound proteins were eluted with SDS sample buffer and the eluates were transferred to a nitrocellulose membrane. The amount of PI3K gamma was determined with the Odyssey Infrared Imaging system.
   7A: Dot blot probed with antibodies and signals detected with Odyssey infrared imaging system. First antibody: anti-PI3K gamma (Jena Bioscience ABD-026S; mouse antibody). Second antibody: anti-mouse IRDye800 (Rockland, 610-732-124).
   7B: Competion binding curves. Relative Odyssey units (Integrated Intensity; integrated kilopixel/mm²) are plotted against compound concentrations and half maximal binding competition (IC) values calculated. Compound 1 (LY294002): IC₅₀ > 30 µM; compound 2 (AS-605240): IC₅₀ = 4.6 µM; compound 3 (AS-604850): IC₅₀ =176 nM.

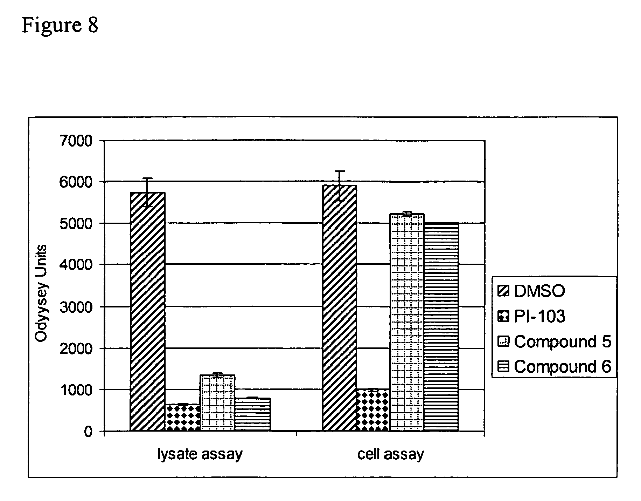

**Figure 8****:** Compound profiling by adding compounds to cell lysates (lysate assay) or by incubating compound with living RAW264.7 cells (cell assay).
   The experiment was performed as described in example 5. Compounds were used at a concentration of 10 µM in both assays and the amount of PI3Kdelta was quantified with the Odyssey Infrared Imaging system.

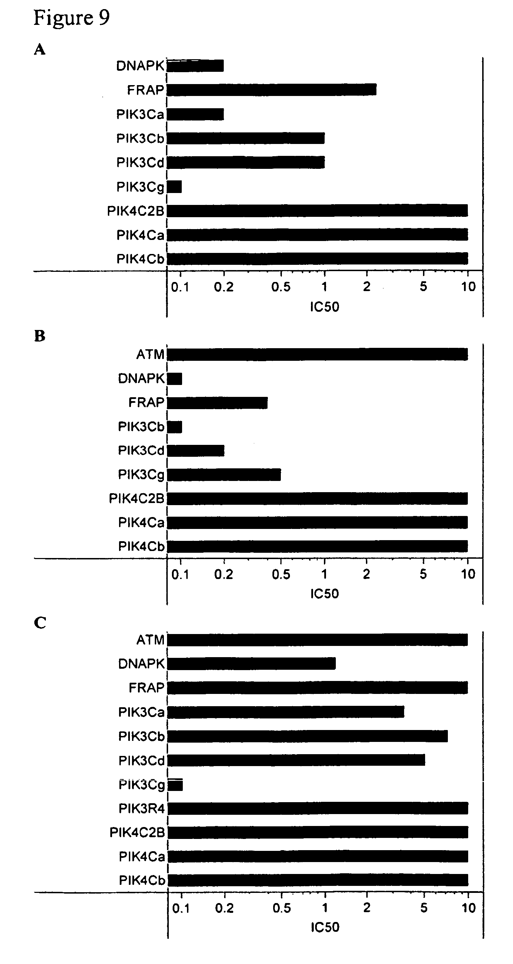

**Figure 9****:** Selectivity profiling of PI3K inhibitors using mass spectrometry quantification. The experiment was performed as a Kinobeads competition binding assay in Ramos cell lysates as described in Example 6. Based on quantitative mass spectrometry profiling the IC₅₀ values (µM) are shown for individual targets.
   A: Compound CZC00015097
   B: Compound CZC00018052
   C: Compound CZC00019091
**Figure 10****:** Dose response curves of compound CZC18052.
   The compound was tested in the competition binding assay with multiplexed immunodetection of kinases as described in Example 7. In a single assay, the binding affinity of the compound was measured for PI3Kalpha, PI3Kbeta, PI3Kgamma, PI3Kdelta and DNAPK. Briefly, a 1:1 mixture of Molt-4 and Jurkat cell lysates was incubated with the affinity matrix (1:1 mixture of beads with immobilized phenylthiazole ligand 1 and beads with the phenylmorpholin-chromen ligand) and compound CZC18052. The beads were washed and the bound kinases were eluted. Aliquots of the eluate were spotted on five different nitrocellulose membranes, each of which was incubated with the respective target antibody and subsequently a fluorescent secondary antibody. The fluorescent signal was quantified using an infrared scanner. The compound showed potent binding for a range of kinases:
   PI3Kalpha (IC₅₀ = 0.027 µM), PI3Kbeta (IC₅₀ = 0.034 µM), PI3Kgamma (IC₅₀ = 0.43 µM), PI3Kdelta (IC₅₀ = 0.14 µM) and DNAPK (IC₅₀ = 0.038 µM).

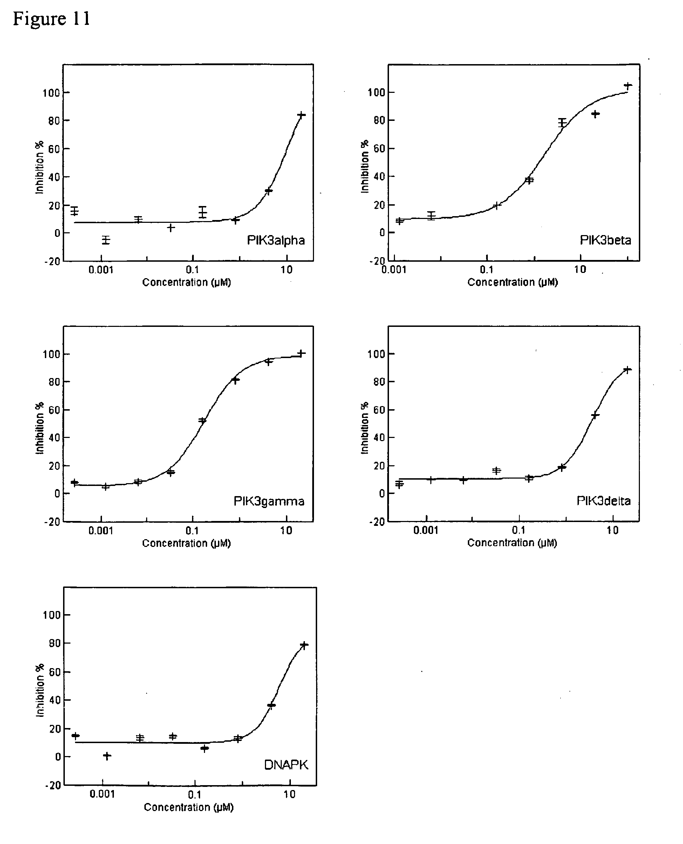

**Figure 11****:** Dose response curves for compound CZC19950.
   The experiment was carried out as described in Example 7. The compound showed binding to the following kinases: PI3Kalpha (IC₅₀ > 7 µM), PI3Kbeta (IC₅₀ = 1.7 µM), PI3Kgamma (IC₅₀ = 0.17 µM). PI3Kdelta (IC₅₀ > 3 µM) and DNAPK (IC₅₀ > 6 µM). Compound CZC19950 showed potent binding only to PI3Kgamma (IC₅₀ = 0.17 µM).

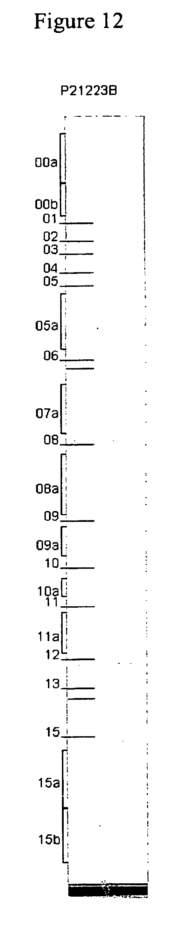

**Figure 12****:** Drug pulldown experiment with immobilized phenylthiazole ligand 1 for mass spectrometry analysis of proteins.
   A protein gel after staining with Coomassie blue is shown. The indicated gel areas were cut out as gel slices and proteins were subjected to analysis by mass spectrometry. The drug pulldown experiment was performed as described in Example 2 with a 1:1 mixture of Jurkat and Ramos cell lysate sample containing 50 mg of protein. Proteins bound to immobilized phenylthiazole ligand 1 were eluted with SDS sample buffer and separated by SDS-polyacrylamide gel electrophoresis (SDS-PAGE). The following proteins were identified in this experiment: DNA-PK, ATM, and mTOR.

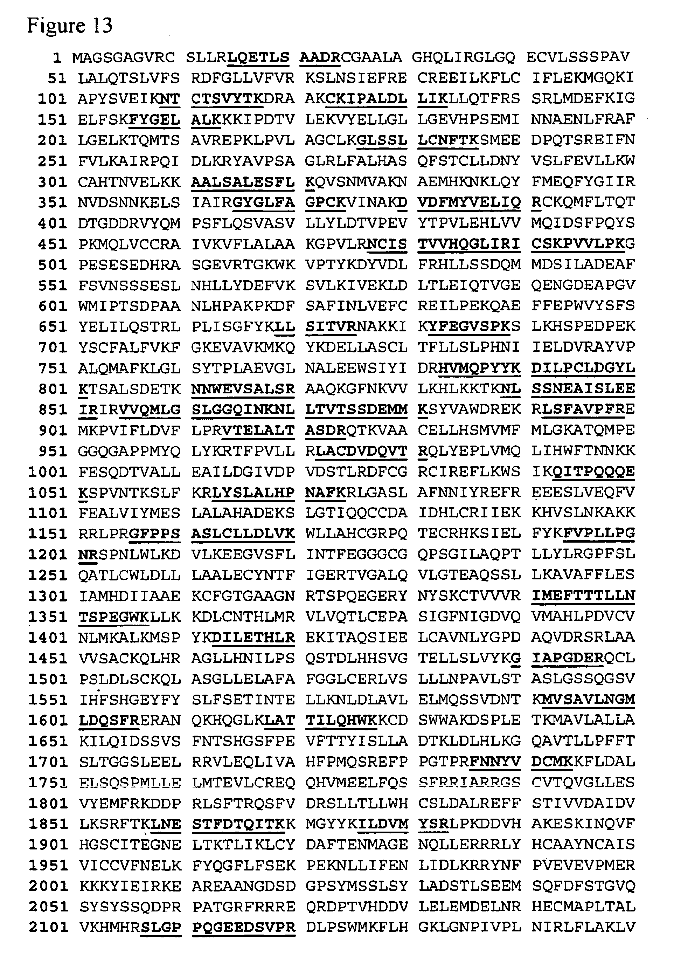

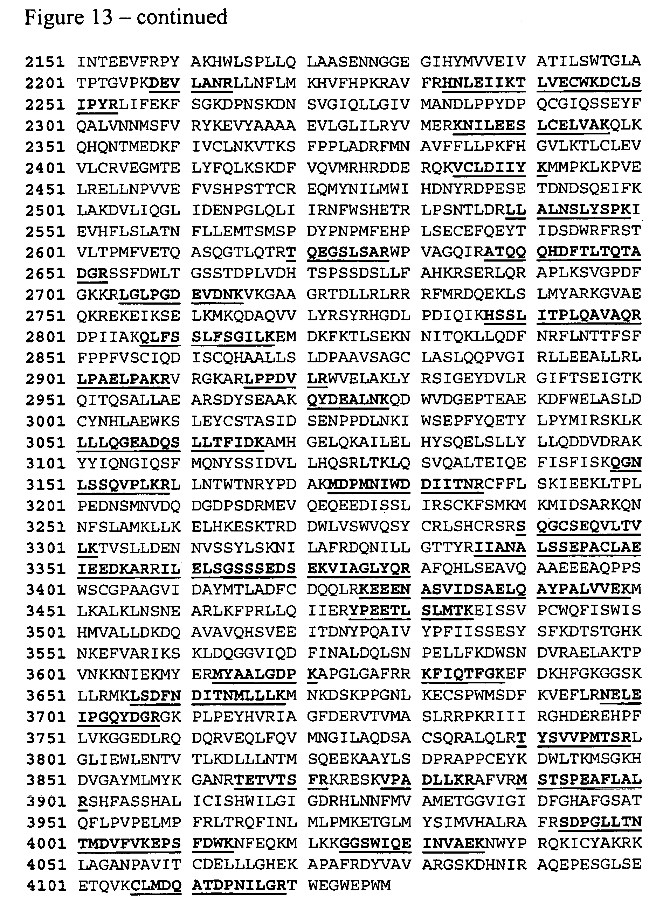

**Figure 13****:** Peptides identified by mass spectrometry of DNA-PK after a drug pulldown with immobilized phenylthiazole ligand 1. The identified peptides are underlined.

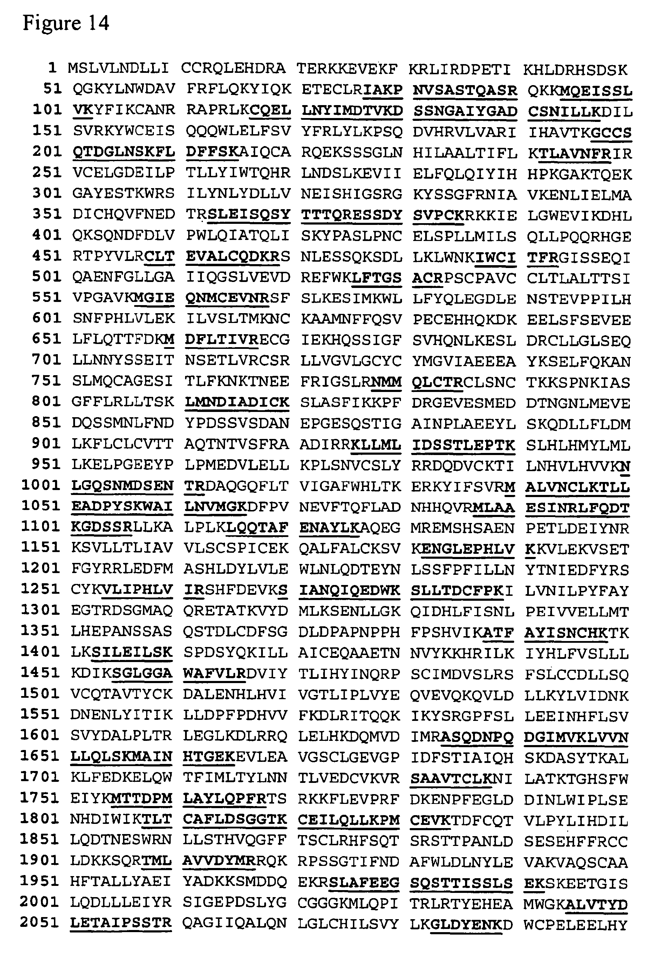

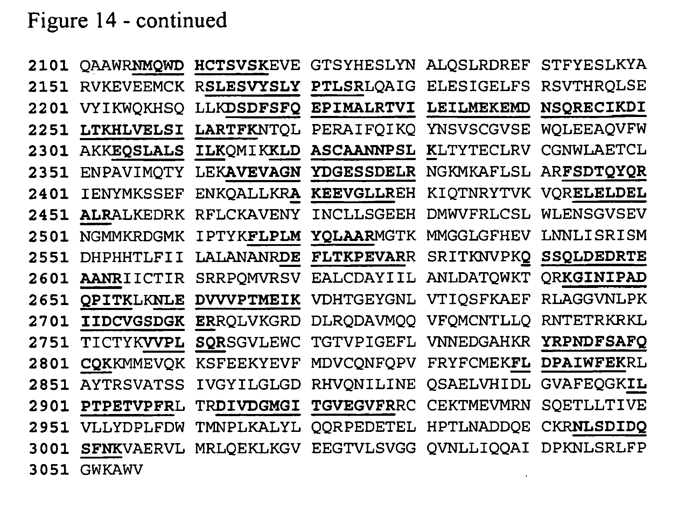

**Figure 14****:** Peptides identified by mass spectrometry of ATM after a drug pulldown with immobilized phenylthiazole ligand 1.

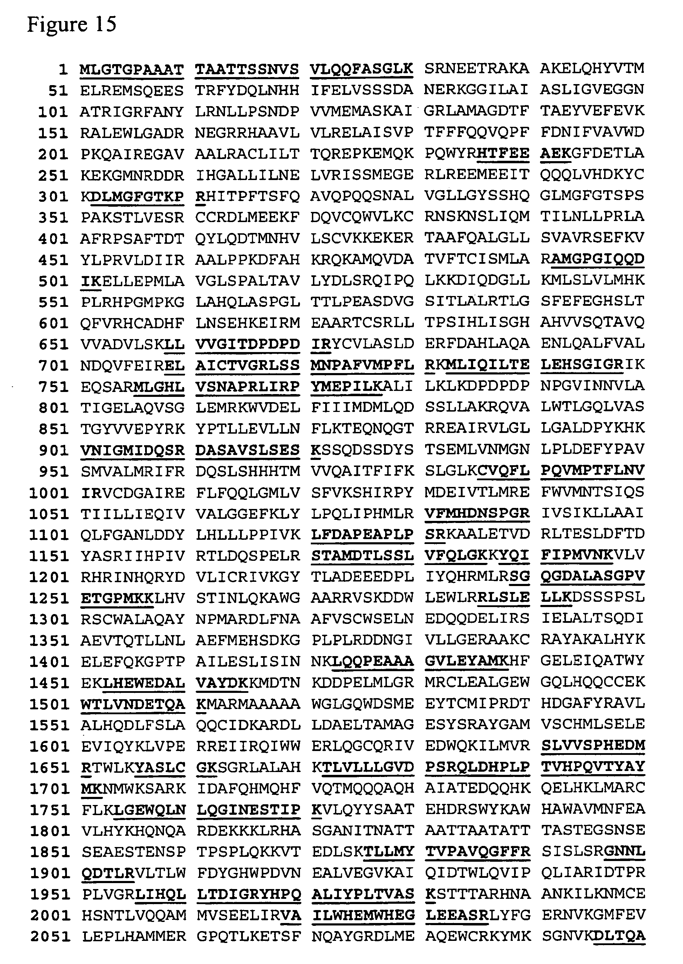

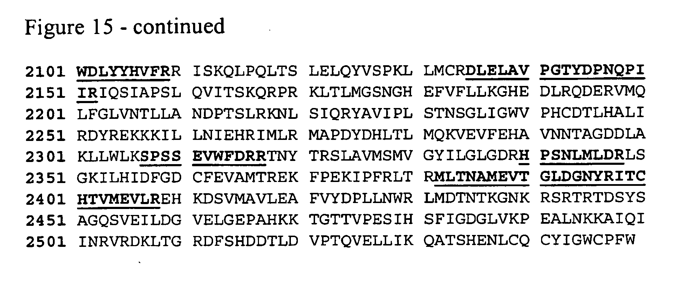

**Figure 15****:** Peptides identified by mass spectrometry of mTOR after a drug pulldown with immobilized phenylthiazole ligand 1.
**Figure 16****:** Synthesis and structure of the phenylmorpholin-chromen ligand (8-(4-aminomethyl-phenyl)-2-morpholin-4-yl-chromen-4-one). The ligand was synthesized as described in Example 8. The structure of the ligand is shown [G].

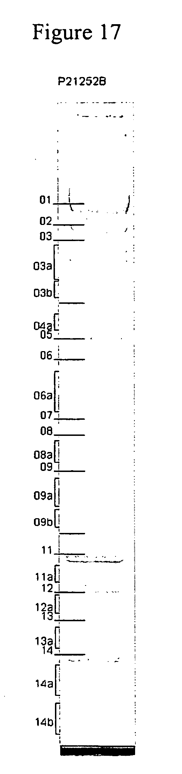

**Figure 17****:** Drug pulldown experiment with the immobilized phenylmorpholin-chromen ligand for mass spectrometry analysis of proteins.
   A protein gel after staining with Coomassie blue is shown. The indicated gel areas were cut out as gel slices and proteins were subjected to analysis by mass spectrometry. The drug pulldown experiment was performed as described in Example 2 with a 1:1 mixture of HeLa and K-562 cell lysate sample containing 50 mg of protein. Proteins bound to the phenylmorpholin-chromen ligand were eluted with SDS sample buffer and separated by SDS-polyacrylamide gel electrophoresis (SDS-PAGE).

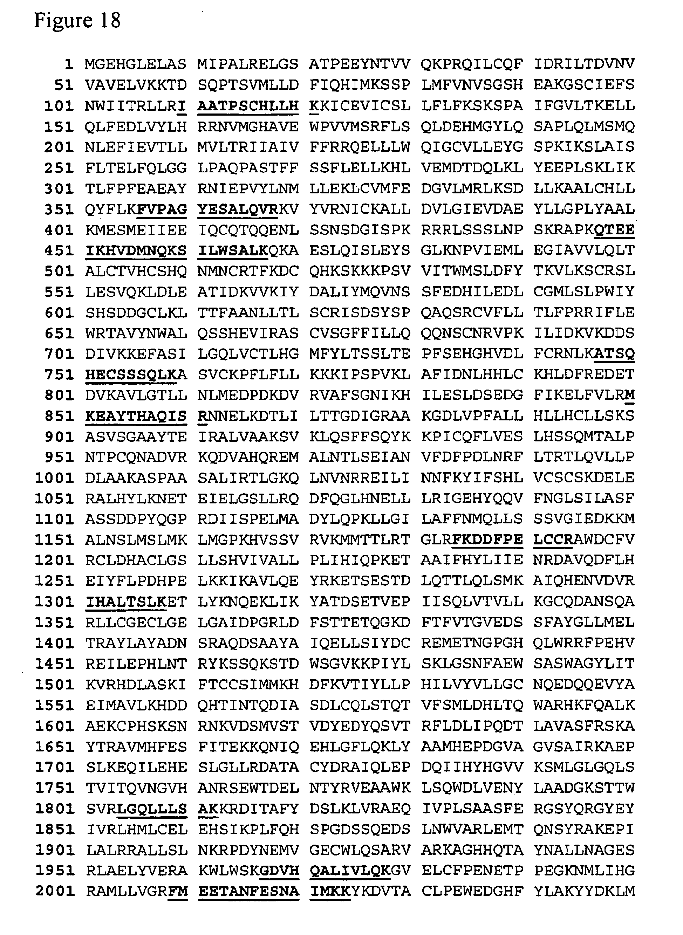

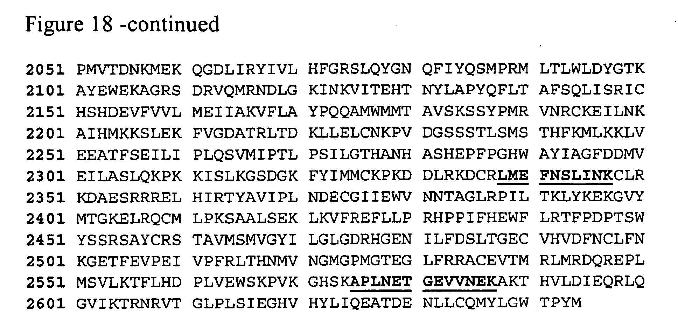

**Figure 18****:** Peptides identified by mass spectrometry of human AT after a drug pulldown with the immobilized phenylmorpholin-chromen ligand from HeLa - K562 lysate mix.

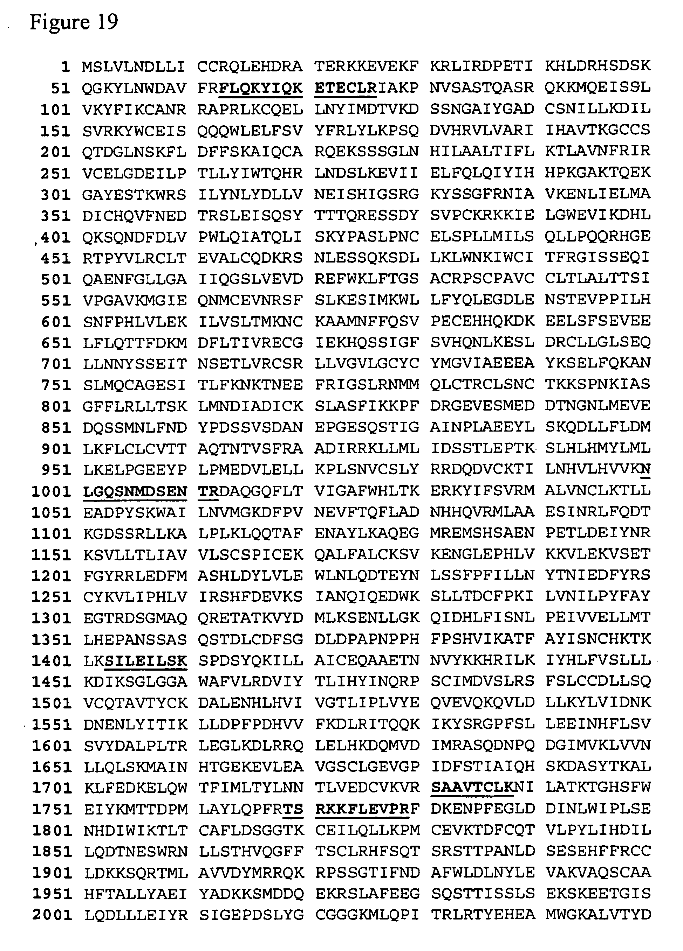

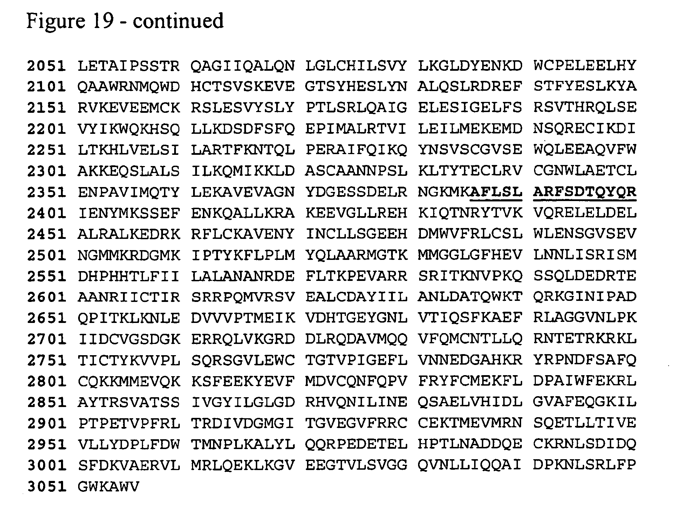

**Figure 19****:** Peptides identified by mass spectrometry of human ATM after a drug pulldown with the immobilized phenylmorpholin-chromen ligand from HeLa - K562 lysate mix.

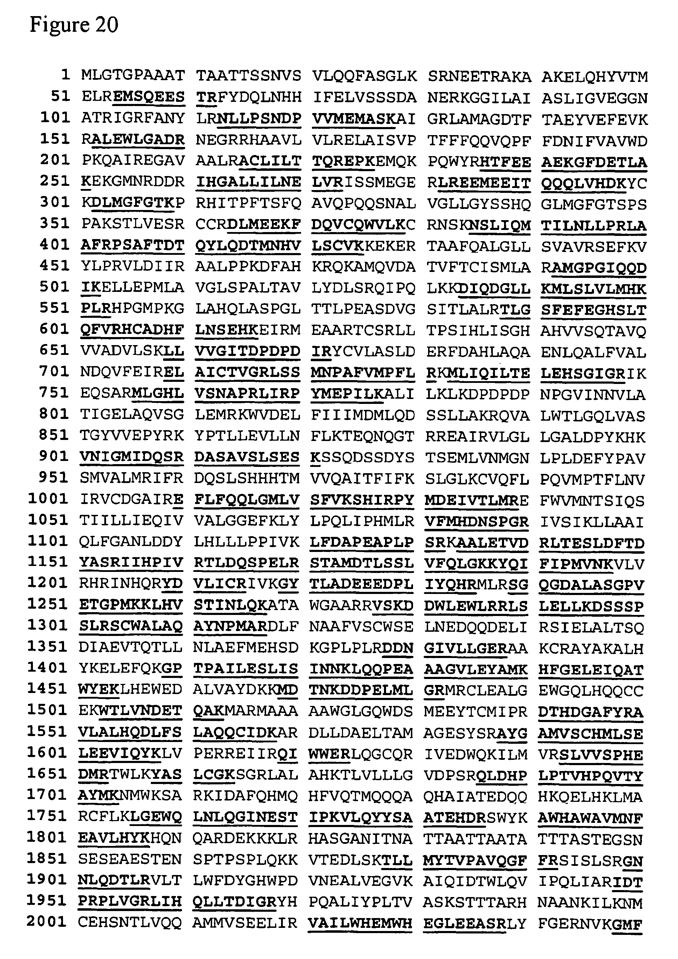

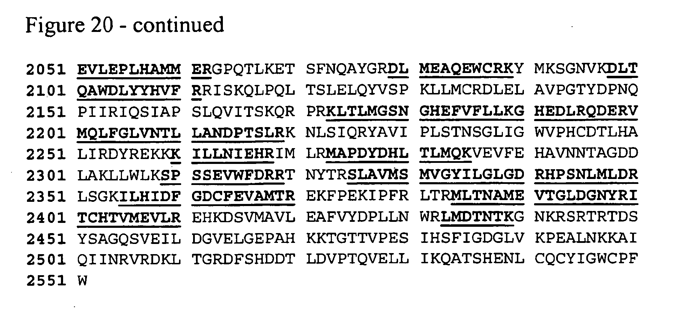

**Figure 20****:** Peptides identified by mas spectrometry of human mTOR after a drug pulldown with the immobilized phenylmorpholin-chromen ligand from HeLa - K562 lysate mix.

### Example 1: Preparation of the affinity matrix

This example illustrates the preparation of the affinity matrix for affinity capture of PI3K kinases from cell lysates. The capturing ligand was covalently immobilized on a solid support through covalent linkage using an amino functional group. This affinity matrix was used in example 2, example 3 and example 4.

### Synthesis of a phenylthiazole ligand 1 (3-(2-{2-[2-(2-amino-etboxy)-etboxy]-ethoxy}-ethoxyrN-[5-(4-chloro-3-methanesulfonyl-phenyl)4-methyl-thiazol-2yl]-propionamide hydrochloride salt)

Steps 1-3: 1-bromo-1-(4-chloro-3-methanesulfonyl-phenyl)-propan-2-one was prepared following the procedure described in WO 2003/072557.

Step 4:5-(4-chloro-3-methanesufhnyl-phenyl)-4-methyl-thiazol-2-ylamine

1-bromo-1-(4-chloro-3-methanesulfhnyl-phenyl)-propan-2-one (480mg 1.5nmol) and thiourea (114mg 1.5mmol) were combined in ethanol (12ml) and heated to 70°C for 2 hours. The reaction mixture was allowed to cool to room temperature and the solid product was collected by filtration and dried under vacuum to yield 5-(4-chloro-3-methanesufonyl-phenyl)-4-methyl-thiazol-2-ylamine as an off-white solid (375mg). ¹H NMR (400MHz DMSO-*d*₆) δ 9.4 (br s, 2H), 8.0 (d, 1H), 7.9 (d, 1H), 7.8 (dd, 1M), 3.4 (s, 3H), 2.3 (s, 3H).

Step 5: (2-{2-[2-(2-{2[5-(4-chloro-3-methanesulfonyl-phenyl)-4-methyl-thiazol-2-ylcarbamoyl]-ethoxy}-ethoxy)-ethoxy]-ethoxy}-ethy]-carbamic acid tert-butyl ester 3-(2-{2-[2-(2-tert-butoxycarbonylamino-ethoxy)-ethoxy]-ethoxy}-ethoxy)-propionic acid (690mg 1.9mmol), EDAC (403mg 2.1mmol), HOBT (284mg 2.1mmol), NMM (420uL 3.8mmol) and 5-(4-chloro-3-methanesufonyl-pheny)-4-methyl-thiazol-2-yiamine (520mg 1.7mmol) were combined in dimethylformamide (16ml) and stirred over night at room temperature. The solvent was removed under reduced pressure and the residue dissolved in dichloromethane (150ml), washed with 1M HCl aqueous solution (50ml) and saturated aqueous sodium hydrogen carbonate (50ml),dried (Magnesium sulphate), filtered and evaporated. The residue was purified by flash chromatography using 50g IST silica flash cartridge eluting with 0-2% methanol/dichloromethane to yield (2-{2-[2-(2-{2[5-(4-chloro-3-methanesulfonyl-phenyl)-4-methyl-thiazol-2-ylcarbamoyl]-ethoxy}-ethoxy)-ethoxy]-ethoxy}-ethyl)-carbamic acid tert-butyl ester as an oil (1.1g residual solvent present) ¹H NMR (400MHz CDCl3) 10.3 (br s, 1H), 8.2 (s, 1H), 7.6 (m, 2H), 7.2 (br s, 1H), 3.9 (t, 2H) 3.8-3.5 (br m, 14H), 3.3 (br m, 5H), 2.8 (t, 2H), 2.4 (s, 3H), 1.4 (s, 9H).

Step 6:3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide hydrochloride

(2-{2-[2-(2-{2[5-(4-chloro-3-rnethanesulfonyl-phenyl)-4-methyl-thiazol-2-ylcarbamoyl]-ethoxy}-ethoxy)-ethoxy]-ethoxy}-ethyl)-carbamic acid tert-butyl ester (1.0g 1.5mmol) was dissolved in dichloromethane (10ml) and treated with HCl (4ml 4M solution in dioxane). The reaction was stirred at room temperature for 3 hours. The solvent was evaporated and the residue dried under vacuum to yield 3-(2-{2-[2-(2-amino-ethoxy)-ethoxy]-ethoxy}-ethoxy)-N-[5-(4-chloro-3-methanesuIfonyl-phenyl)-4-methyl-thiazol-2yl]-propionamide hydrochloride as a yellow viscous oil ( 830mg residual solvent present) ¹H NMR (400MHz CDCl3) 8.4 (br s, 3H), 8.2 (s, 1H), 7.7 (br d, 1H), 7.6 (br d, 1H) 3.9 (br m, 4H), 3.8-3.6 (br m, 12H), 3.3 (s, 3H), 3.3 (br m, 2H), 3.1 (br m, 2H), 2.6 (s, 3H). NMR spectra were obtained on a Bruker dpx400.

**Table 1: Abbreviations used**

| | |
|---|---|
| br | broad |
| CDCl3 | deuterochloroform |
| d | doublet |
| dd | doublet of doublets |
| DMSO | dimethyl sulphoxide |
| EDAC | 1-ethyl-3-(3'-dimethylarninopropyl)carbodiinide |
| g | gram |
| HCl | Hydrochloric acid |
| HOBT | N-Hydroxybenzotriazole |
| m | multiplet |
| mg | milligram |
| ml | millilitre |
| mmol | millimole |
| M | molar |
| MHz | megahertz |
| NMM | N-methyl morpholine |
| NMR | nuclear magnetic resonance |
| q | quartet |
| s | singlet |
| t | triplet |

### Immobilization of phenylthiazole ligand 1 on beads (affinity matrix)

NHS-activated Sepharose 4 Fast Flow (Amersham Biosciences, 17-0906-01) was equilibrated with anhydrous DMSO (Dimethylsulfoxid, Fluka, 41648, H20 <= 0.005%). 1 ml of settled beads was placed in a 15 ml Falcon tube, compound stock solution (usually 100 mM in DMF or DMSO) was added (final concentration 0.2-2 µmol/ml beads) as well as 15 µl of triethylamine (Sigma, T-0886, 99% pure). Beads were incubated at room temperature in darkness on an end-over-end shaker (Roto Shake Genie, Scientific Industries Inc.) for 16 - 20 hours. Coupling efficiency is determined by HPLC. Non-reacted NHS-groups were blocked by incubation with aminoethanol at room temperature on the end-over-end shaker over night. Beads were washed with 10 ml of DMSO and were stored in isopropanol at -20°C. These beads were used as the affinity matrix in example 2, 3 and 4. Control beads (no ligand immobilized) were generated by blocking the NHS-groups by incubation with aminoethanol as described above.

### Example 2: Drug pulldown of PI3K using immobilized phenylthiazole ligand 1

This example demonstrates the use of the immobilized phenylthiazole ligand 1 for the identification of PI3K proteins from cell lysates of a human T cell line (MOLT4 cells; ATCC number CRL-1582). To this end a lysate of MOLT-4 cells was contacted with the affinity matrix described in example 1. Proteins binding to the phenylthiazole ligand 1 were identified by Western blot and mass spectrometry (MS) analysis.
For Western blot analysis bound proteins were eluted from the affinity matrix and subsequently separated by SDS-Polyacrylamide gel elecrophoresis. PI3K gamma and PI3K delta were detected with specific antibodies (Figure 2). The results of the Western blot analysis show that immobilized phenylthiazole ligand 1 captures (pulls down) PI3K gamma and PI3K delta from the cell lysate.

For the identification of proteins by mass spectrometry analysis the proteins captured by the affinty matrix were eluted and subsequently separated by SDS-Polyacrylamide gel elecrophoresis (Figure 3). Suitable gel bands were cut out and subjected to in-gel proteolytic digestion with trypsin and analyzed by LC-MS/MS mass spectrometry.

The identification of members of the PI3K family is documented in Table 3. The peptide sequence coverage of PI3K gamma is shown in Figure 4 and for PI3K delta in Figure 5.

### 1. Cell culture

MOLT-4 cells (ATCC number 1582) were grown in 1 litre Spinner flasks (Integra Biosciences, #182101) in suspension in RPMI 1640 medium (Invitrogen, #21875-034) supplemented with 10% Fetal Bovine Serum (Invitrogen) at a density between 0.15 x 10⁶ and 1.2 x 10⁶ cells/ml. Cells were harvested by centrifugation, washed once with 1 x PBS buffer (Invitrogen, #14190-094) and cell pellets were frozen in liquid nitrogen and subsequently stored at -80°C.

### 2. Preparation of cell lysates

MOLT-4 cells were homogenized in a Potter S homogenizer in lysis buffer: 50 mM Tris-HCl, 0.8% NP40, 5% glycerol, 150 mM NaCl, 1.5 mM MgCl2, 25 mM NaF, 1 mM sodium vanadate, 1 mM DTT, pH 7.5. One complete EDTA-free tablet (protease inhibitor cocktail, Roche Diagnostics, 1 873 580) per 25 ml buffer was added. The material was dounced 10 times using a mechanized POTTER S, transferred to 50 ml falcon tubes, incubated for 30 minutes on ice and spun down for 10 min at 20,000 g at 4°C (10,000 rpm in Sorvall SLA600, precooled). The supernatant was transferred to an ultracentrifuge (UZ)-polycarbonate tube (Beckmann, 355654) and spun for 1 hour at 100.000 g at 4°C (33.500 rpm in Ti50.2, precooled). The supernatant was transferred again to a fresh 50 ml falcon tube, the protein concentration was determined by a Bradford assay (BioRad) and samples containing 50 mg of protein per aliquot were prepared. The samples were immediately used for experiments or frozen in liquid nitrogen and stored frozen at -80°C.

### 3. Compound pull-down experiment

Sepharose-beads with immobilized compound (100 µl beads per pull-down experiment) were equilibrated in lysis buffer and incubated with a cell lysate sample containing 50 mg of protein on an end-over-end shaker (Roto Shake Genie, Scientific Industries Inc.) for 2 hours at 4°C. Beads were collected, transfered to Mobicol-columns (MoBiTech 10055) and washed with 10 ml lysis buffer containing 0.5% NP40 detergent, followed by 5 ml lysis buffer with 0.25 % detergent. To elute the bound protein, 60 µl 2 x SDS sample buffer was added, the column was heated for 30 minutes at 50°C and the eluate was transferred to a microfuge tube by centrifugation. Proteins were then separated by SDS-Polyacrylamide electrophoresis (SDS-PAGE).

### 4. Protein detection by Western blot analysis

Western blots were performed according to standard procedures and the PI3K proteins were detected and quantified by using specific anti-PI3K antibodies (first antibody), a fluorescently labeled secondary antibody and the Odyssey Infrared Imaging system from LI-COR Biosciences (Lincoln, Nebraska, USA) according to instructions provided by the manufacturer (Schutz-Geschwendener et al., 2004. Quantitative, two-color Western blot detection with infrared fluorescence. Published May 2004 by LI-COR Biosciences, www.licor.com).

The mouse anti PI3K gamma antibody (Jena Bioscience, catalogue number ABD-026S) was used at a dilution of 1:200 and incubated with the blot over night at 4°C. The secondary anti-mouse IRDye^{™} 800 antibody (Rockland, ctalogue number 610-732-124) was used at a dilution of 1:15000. The rabbit anti PI3K delta antibody (Santa Cruz, catalogue number sc-7176 was diluted 1:600 and incubated over night at 4°C. As a secondary detection antibody the anti-rabbit IRDyeTM 800 antibody was diluted 1:20 000 (LICOR, catalogue number 926-32211).

### 5. Protein Identification by Mass Spectrometry

### 5.1 Protein digestion prior to mass spectrometric analysis

Gel-separated proteins were reduced, alkylated and digested in gel essentially following the procedure described by Shevchenko et al., 1996, Anal. Chem. 68:850-858. Briefly, gel-separated proteins were excised from the gel using a clean scalpel, reduced using 10 mM DTT (in 5mM ammonium bicarbonate, 54°C, 45 min) and subsequently alkylated with 55 mM iodoacetamid (in 5 mM ammonium bicarbonate) at room temperature in the dark (30 minutes). Reduced and alkylated proteins were digested in gel with porcine trypsin (Promega) at a protease concentration of 12.5 ng/µl in 5mM ammonium bicarbonate. Digestion was allowed to proceed for 4 hours at 37°C and the reaction was subsequently stopped using 5 µl 5% formic acid.

### 5.2 Sample preparation prior to analysis by mass spectrometry

Gel plugs were extracted twice with 20 µl 1% TFA and pooled with acidified digest supernatants. Samples were dried in a a vaccuum centrifuge and resuspended in 10 µl 0.1% formic acid.

### 5.3. Mass spectrometric data acquisition

Peptide samples were injected into a nano LC system (CapLC, Waters or Ultimate, Dionex) which was directly coupled either to a quadrupole TOF (QTOF2, QTOF Ultima, QTOF Micro, Micromass) or ion trap (LCQ Deca XP) mass spectrometer. Peptides were separated on the LC system using a gradient of aqueous and organic solvents (see below). Solvent A was 5% acetonitrile in 0.5% formic acid and solvent B was 70% acetonitrile in 0.5% formic acid.

**Table 2: Peptides eluting off the LC system were partially sequenced within the mass spectrometer.**

| Time (min) | % solvent A | % solvent B |
|---|---|---|
| 0 | 95 | 5 |
| 5.33 | 92 | 8 |
| 35 | 50 | 50 |
| 36 | 20 | 80 |
| 40 | 20 | 80 |
| 41 | 95 | 5 |
| 50 | 95 | 5 |

### 5.4. Protein identification

The peptide mass and fragmentation data generated in the LC-MS/MS experiments were used to query fasta formatted protein and nucleotide sequence databases maintained and updated regularly at the NCBI (for the NCBInr, dbEST and the human and mouse genomes) and European Bioinformatics Institute (EBI, for the human, mouse, D. melanogaster and C. elegans proteome databases). Proteins were identified by correlating the measured peptide mass and fragmentation data with the same data computed from the entries in the database using the software tool Mascot (Matrix Science; Perkins et al., 1999. Probability-based protein identification by searching sequence databases using mass spectrometry data. Electrophoresis 20, 3551-3567). Search criteria varied depending on which mass spectrometer was used for the analysis.

**Table 3: PI3K proteins identified by mass spectrometry (MOLT-4 cells; experiment P15234B; MS sample refers to the gel slice cut out from the polyacrylamide gel (Figure 3).**

| **MS sample** | **Protein accesion number (IPI)** | **Protein name** | **Number of peptides identified** |
|---|---|---|---|
| 4 | IPI00070943.3 | PIK4CA; phosphatidylinositol 4-kinase, catalytic, alpha polypeptide | 62 |
| 5 | PI00024006.1 | PIK3R4; phosphoinositide-3-kinase, regulator subunit 4, p150 | 11 |
| 6 | IPI00292690.1 | PIK3CG; phosphoinositide-3-kinase, catalytic, gamma polypeptide | 39 |
| 6 | IPI00298410.2 | PIK3CD; phosphoinositide-3-kinase, catalytic, delta polypeptide | 26 |
| 7 | IPI00298410.2 | PIK3CD; phosphoinositide-3-kinase, catalytic, delta polypeptide | 12 |
| 8 | IP100002591.3 | PIK4CB; phosphatidylinositol 4-kinase, catalytic, beta polypeptide | 15 |
| 9 | IPI00021448.1 | PIK3R1; phosphoinositide-3-kinase, regulatory subunit 1 (p85 alpha) | 27 |
| 9 | IPI00011736.3 | PIK3R2; phosphoinositide-3-kinase, regulatory subunit 2 (p85 beta) | 8 |
| 14 | IPI00333040.3 | PIK3R1; phosphoinositide-3-kinase, regulatory subunit 1 (p85 alpha) | 7 |

### Example 3: Elution assay for the identification of PI3K gamma interacting compounds

The preparation of the phenylthiazole ligand 1 affinity matrix was done as described in example 1. To screen maximally 80 compounds in a 96 well plate the elution experiment is performed as described below.

### Elution assay

The affinity matrix (1200 µl of beads) was washed 2 x with 30 ml 1x DP-buffer. After each washing step the beads were collected by centrifugation for 2 minutes at 1200 rpm at 4°C in a Heraeus centrifuge. The supernatants were discarded. Finally, the beads were equilibrated in 15 ml binding buffer (1x DP buffer/0.4% NP40). After this incubation time the beads were harvested and mixed in a 50 ml falcon tube with MOLT-4 cell lysate at a protein concentration of 5 mg/ml with a total amount of 75mg protein. The preparation of the lysate was done as described in example 2. Beads and the lysate were incubated for 2 hours at 4°C. After the incubation with the lysate beads were collected by centrifugation as described and transferred to 2 ml columns (MoBiTec, #S10129) and washed with 10 ml 1x DP buffer/0.4% NP40 and 5ml 1x DP buffer/0.2% NP40. Once the washing buffer had run through the column completely the volume of beads left in the column was calculated (approximately 1000 µl). The beads were resuspended in 4 fold excess of 1x DP-buffer/0.2% NP40 (4 ml) to generate a 20% slurry. For compound elution tests 50 µl of this suspension was added to each well of a 96 well plate (Millipore MultiScreenHTS, MSBVN1210, with lid and 1.2um hydrophilic low protein binding Durapore membrane). To remove residual buffer the 96 well plate was assembled with Assemble filter and collection plate and this sandwich assembly was spun down for 10 seconds at 800 rpm in a centrifuge. Then 40 µl of elution buffer (1x DP-buffer/0.2% NP40) supplemented with the test compound was added to the beads. Test compounds were prepared by diluting them in dilution buffer starting from 40 fold concentrated stock solution in DMSO. The plate was assembled on the collection plate, fixed on an Eppendorf incubator and incubated for 30 minutes at 4°C at 650 rpm shaking. To harvest the eluate the 96 well filter plate assembled on the 96 well collection plate was centrifuged for 1 minute at 800 rpm in a table top centrifuge at 4°C (Heraeus). The eluates were checked for the presence of PI3Kgamma and PI3Kdelta by using a dot blot procedure.

### Detection of eluted PI3K gamma

The eluted PI3K gamma protein was detected and quantified by a dot blot procedure using an antibody directed against PI3K gamma (Jena Bioscience, # ABD-026S), a fluorescently labeled secondary anti mouse IRDye™ 800 (Rockland, #610-732-124) and the Odyssey Infrared Imaging system from LI-COR Biosciences (Lincoln, Nebraska, USA) according to instructions provided by the manufacturer (Schutz-Geschwendener et al., 2004. Quantitative, two-color Western blot detection with infrared fluorescence. Published May 2004 by LI-COR Biosciences, www.licor.com).

Nitrocellulose membranes were treated with 20% ethanol and subsequently washed with 1 x PBS buffer. Eluates (as described above) were combined with 12 µl of 4 x SDS loading buffer (200 mM Tris-HCl pH6.8, 8% SDS, 40% glycerol, 0.04% Bromphenol blue) and applied to the Nitrocellulose membrane with a BioRad dot blot appartus (BioRad, #170-6545).

For detection of PI3K gamma the membranes were first blocked by incubation with Odyssey blocking buffer for 1 hour. Blocked membranes were incubated for 16 hours at 4°C with the first antibody (mouse anti PI3K gamma from Jena Bioscience, ABD-026S) diluted 1:100 in Odyssey blocking buffer supplemented with 0.2% Tween 20. After washing the membrane four times for 5 minutes with 1 x PBS buffer containing 0.1% Tween 20 the membrane was incubated for 40 minutes with the detection antibody (anti-mouse IRDye^{™} 800 from Rockland, 610-732-124) , diluted 1:10 000 in Odyssey Blocking Buffer supplemented with 0.2% Tween 20. Afterwards the membrane was washed four times for 5 minutes with 1 x PBS buffer/0.1% Tween 20 and once for 5 minutes with 1 x PBS buffer. Afterwards the membrane was scanned with the Odyssey reader and data were analysed.

**Table 4: Preparation of 5x-DP buffer**

| **Substance:** | **Stock solution** | **Final conc. in 1 x lysis buffer** | **Add for 115 x lysis buffer** |
|---|---|---|---|
| **Tris/-HCI pH 7.5** | 1 M | 50 mM | 250 ml |
| **Glycerol** | 87 % | 5% | 288 ml |
| **MgCl₂** | 1M | 1.5 mM | 7.5 ml |
| **NaCl** | 5 M | 150 mM | 150 ml |
| **Na₃VO₄** | 100 mM | 1 mM | 50 ml |

The 5x-DP buffer was filtered through 0.22 µm filter and stored in 40 ml-aliquots at - 80°C. These solutions were obtained from the following suppliers: 1.0 M Tris/HCl pH 7.5 (Sigma, T-2663), 87% Glycerol (Merck, catalogue number 04091.2500); 1.0 M MgCl₂ (Sigma, M-1028); 5.0 M NaCl (Sigma, S-5150).

### Test compounds for elution

The test compounds listed below were used for elution experiments after dilution as described below. Typically all compounds were dissolved in 100 % DMSO (Fluka, cat.no 41647) at a concentration of 100 mM or 50 mM. Compounds are stored at -20°C. Dilution of test compound for elution experiments: Prepare 50 mM stock by diluting the 100 mM stock 1:1 with 100% DMSO. For elution experiments further dilute the compound with elution buffer (1x DP-buffer/0.2% NP40). Compounds used for elution:
Compound 1: PI3K inhibitor LY294002 (Tocris 1130; Vlahos et al., 1994, J. Biol. Chem. 269, 5241-5248).
Compound 2: PI3K gamma inhibitor (Calbiochem 528106; AS-605240; Camps et al., 2005, Nature Medicine 11, 936-943).
Compound 3: PI3K gamma inhibitor II (Calbiochem 528108; AS-604850; Camps et al., 2005, Nature Medicine 11, 936-943).

### Example 4: Competitive binding assay for the identification of PI3K gamma interacting compounds

This examples demonstrates a competitive binding assay in which test compunds are added directly into a cell lysate. Test compounds (at various concentrations) and the affinity matrix with the immobilzed phenylthiazole ligand 1 were added to lysate aliquots and allowed to bind to the proteins contained in the lysate sample. After the incubation time the beads with captured proteins were separated from the lysate. Bound proteins were then eluted and the presence of PI3K gamma was detected and quantified using a specific antibody in a dot blot procedure and the Odyssey infrared detection system (Figure 7A). Dose response curves for three compounds were generated (Figure 7B).

### Washing of affinity matrix

The affinity matrix as described in example 1 (1.1 ml of dry volume) was washed two times with 15 ml of 1x DP buffer containing 0.4% NP40 and then resupended in 5.5 ml of 1x DP buffer containing 0.4% NP40 (20% beads slurry).

### Preparation of test compounds

Stock solutions of test compounds were prepared in DMSO corresponding to a 100fold higher concentration compared to the final desired test concentraion (e.g. a 4 mM stock solution was prepared for a final test concentration of 4 µM). This dilution scheme resulted in a final DMSO concentration of 1%. For control experiments (no test compound) a buffer containing 1% DMSO was used so that all test samples contained 1% DMSO.
Compound 1: PI3K inhibitor LY294002 (Tocris 1130; Vlahos et al., 1994, J. Biol. Chem. 269,5241-5248).
Compound 3: PI3K gamma inhibitor II (Calbiochem 528108; AS-604850; Camps et al., 2005, Nature Medicine 11, 936-943).
Compound 4 (CZC00015387).

### Dilution of cell lysate

Cell lysates were prepared as described in example 2. For a typical experiment 1 lysate aliquot containing 50 mg of protein was thawed in a 37°C water bath and then kept at 4°C. To the lysate one volume of 1xDP buffer was added so that a final NP40 concentration of 0.4% was achieved. Then, 1/50 of the final volume of a 50fold concentrated protease inhibitor solution was added (1 tablet of protease inhibitor dissolved in 0.5 ml of 1x DP buffer containing 0.4% NP40; EDTA-free tablet protease inhibitor cocktail from Roche Diagnostics, catalogue number 41647). The lysate was further dilute by adding 1x DP buffer containing 0.4% NP40 so that a final protein concentration of 5 mg/ml was achieved.

### Incubation of lysate with test compound and affinity matrix

A volume of 100 µl of diluted lysate was dispensed into each well of a 96 well filter plate. Then 1.5 µl of test compound diluted in DMSO was added. For control reactions 1.5 µl DMSO without test compound were used. Then 50 µl of affinity matrix (20% slurry) per well were added. The plate was incubated for 2 hours at 4°C on a shaker (750 rpm on a Thermomixer, Eppendorf).

The plate was washed using a vacuum manifold instrument (Millipore, MAVM 096 0R). Each well was washed 4 times with 400 µl of 1x DP buffer containing 0.4% NP-40 and 2 times with 400 µl with 1x DP buffer containing 0.2% NP-40.

For elution the filter plate was placed on a collection plate and 40 µl of 2x sample buffer (100 mM TrisHCl, pH6.8; 4% SDS; 20% glycerol; 0.02% Bromphenol blue) with DTT (50 mM final concentration) was added to each well. The plates were incubated for 30 minutes at room temperature on a shaker (750 rpm on a Thermomixer, Eppendorf). Subsequently the plates were centrifuged for 2 minutes at 1100 rpm (Heraeus centrifuge) and the eluate was collected in the wells of the collection plate.

### Detection and quantification of eluted PI3K gamma

The PI3K gamma protein in the eluates was detected and quantified by a dot blot procedure using a first antibody directed against PI3K gamma (anti PI3K gamma from Jena Bioscience, ABD-026S) and a fluorescently labeled secondary antibody (anti-mouse IRDye^{™} 800,from Rockland, 610-732-124). The Odyssey Infrared Imaging system from LI-COR Biosciences (Lincoln, Nebraska, USA) was operated according to instructions provided by the manufacturer (Schutz-Geschwendener et al., 2004. Quantitative, two-color Western blot detection with infrared fluorescence. Published May 2004 by LI-COR Biosciences, www.licor.com).

The dot blot apparatus was used according to the instructions of the supplier (Bio-Dot microfiltration apparatus, BioRad 170-65). Nitrocellulose membranes (BioTrace NT Nitrocellulose, PALL BTNT30R) were treated with 20% ethanol and subsequently washed with PBS buffer. Per dot 30 µl of eluate sample were applied and left for 30 min before a vacuum pump was applied.

For detection of PI3K gamma the membranes were first blocked by incubation with Odyssey blocking buffer (LICOR, 927-40000) for 1 hour at room temperature. Blocked membranes were then incubated for 16 hours at 4°C with the first antibody (anti PI3K gamma from Jena Bioscience, ABD-026S) which was diluted in Odyssey blocking buffer containing 0.2% Tween-20. After washing the membrane four times for 5 minutes with PBS buffer containing 0.1% Tween 20 the membrane was incubated for 40 minutes with the detection antibody (anti-mouse IRDys^{™} 800 from Rockland, 610-732-124) diluted in Odyssey blocking buffer containing 0.2% Tween-20. Afterwards the membrane was washed four times for 5 minutes each with 1 x PBS bluffer/0.1% Tween 20 and once for 5 minutes with 1 x PBS buffer. The membrane was kept in PBS buffer at 4°C and then scanned with the Odyssey instrument and signals were recorded and analysed according to the instructions of the manufacturer.

### Example 5: Compound profiling of PI3Kdelta interacting compounds by adding compounds to cell lysates or living cells

This example demonstrates binding assays in which test compounds are added directly into a cell lysate or incubated with living cells (RAW264.7 macrophages).

For the cell lysate competitive binding assay compounds were added to lysate samples and allowed to bind to the proteins contained in the lysate sample. Then the affinity matrix containing the immobilized phenylthiazole ligand was added in order to capture proteins not bound to the test compound. After the incubation time the beads with captured proteins were separated from the lysate by centrifugation. Bead-bound proteins were then eluted and the presence of PI3Kdelta protein was detected and quantified using a specific antibody and the Odyssey infrared detection system.

For the in cell profiling experiment aliquots of life RAW264.7 macrophages were first incubated with compounds for 30 minutes in cell culture medium. During this incubation time the compounds can enter the cells and bind to protein targets within the cells. Then the cells were harvested, cell lysates were prepared and the affinity matrix was added in order to capture proteins not bound to the test compound. After 90 minutes of incubation of the cell lysate with the affinity matrix the beads with the captured proteins were separated from the lysate by centrifugation. Bound proteins were then eluted and the presence of PI3Kdelta was detected and quantified using a specific antibody and the Odyssey infrared detection system.

Both approaches yielded similar results for the cell-permeable reference compound PI-103 (Figure 8). The two other compounds (compound 5 and 6) interacted with PI3Kdelta in the lysate assay but not significantly in the cell assay. A possible reason for this difference is that the latter two compounds were not sufficiently cell-permeable.

### Cell culture

RAW264.7 macrophages (American Type Culture Collection, Rockville, MD) were cultured in Dulbecco's modified Eagle's medium (DMEM, 4mM L-glutamine, 4.5 g/L glucose; Gibco #41965) supplemented with 10 % heat-inactivated fetal bovine serum (Gibco #10270) and 1.5 g/L Sodium bicarbonate (Gibco #25080, 7.5% solution) at 37 °C in a humidified atmosphere in the presence of 5 % CO₂. Macrophages were sub-cultured by scraping the cells from the culture dish in DMEM culture medium using a cell scraper and replating them in fresh culture medium. RAW264.7 macrophages were used for experiments after reaching passage number 3. The cells were washed once with phosphate buffered saline (D-PBS, Gibco #14040), removed from the culture dish in DMEM culture medium and centrifuged at 1,000 rpm at room temperature for 3 minutes. The cell pellet was resuspended in DMEM culture medium and the cell number was determined. 25 x 10⁶ cells were plated onto one 10 cm-culture dish and incubated for 48 hours in fresh DMEM culture medium until they reached approximately 90 % confluence.

### A) Compound profiling in living cells

### Treatment of cells with test compound

The macrophages were washed with D-PBS buffer and fresh DMEM culture-medium was added. Cells were treated with DMEM culture medium containing 0.2 % DMSO (vehicle control) or DMEM culture medium with 10 µM PI-103 (Calbiochem, catalogue number 528100; Knight et al., 2006, Cell 125, 733-747), 10 µM compound 5 or 10 µM compound 6 over a period of 30 minutes. Test compounds were prepared as 20 mM stock solutions in DMSO and further diluted to reach the final concentration of 10 µM compound and 0.2% DMSO in the cell culture medium.

### Preparation of cell lysates

The culture medium was removed, cells were washed once with D-PBS buffer and 4 ml ice-cold D-PBS buffer was added. Macrophages were removed by gently scraping the cells and resuspending them in D-PBS buffer. The cell suspensions were transferred into 15 ml Falcon tubes and kept on ice. The macrophage suspensions were centrifuged at 1500 rpm 4 °C for 3 minutes in a Heraeus Multifuge. The supernatant was removed and the cell pellets were washed with cold D-PBS buffer. After an additional centrifugation step, the cell pellets were quickly frozen in liquid nitrogen. Cells were thawed on ice and lysed by adding 120 µl 1x lysis buffer (1 x DP buffer, 0.8% NP40). The lysates were transferred into 1.5 ml Eppendorf tubes and incubated for 30 minutes rotating at 4 °C and then centrifuged for 10 minutes at 13,200 rpm at 4°C. The supernatants was transferred into ultracentrifuge tubes and centrifuged in a TLA-120.2 rotor at 53,000 rpm (100,000 x g) for 1 hour at 4°C. An aliquot of the clarified supernatant was used for protein quantification performing Bradford assay (Biorad Protein Assay dye concentrate, catalogue number 500-0006). The remaining samples were quickly frozen in liquid nitrogen and stored at -80°C until use in the binding assay.

### Dilution of cell lysate

Cell lysates were prepared as described below from RAW264.7 macrophages. One lysate aliquot was thawed in a 37°C water bath and then kept at 4°C. To the lysate one volume of 1xDP buffer containing protease inhibitor (1 tablet of protease inhibitor dissolved in 25 ml of 1x DP buffer or 25 ml of 1x DP buffer containing 0.8% NP40; EDTA-free tablet protease inhibitor cocktail from Roche Diagnostics, catalogue number 41647) was added so that a final NP40 concentration of 0.8% was achieved. The lysate was further diluted by adding 1x DP buffer containing 0.8% NP40 and proteinase inhibitors so that a final protein concentration of 10 mg/ml was achieved.

### Washing of affinity matrix

The affinity matrix as described in example 1 (0.25 ml of dry bead volume) was washed two times with 10 ml of 1x DP buffer containing 0.2% NP40 and was finally resuspended in 5.0 ml of 1x DP buffer containing 0.2% NP40 (5% beads slurry).

### Incubation of cell lysate with the affinity matrix

A volume of 50 µl of diluted lysate (10 mg/ml protein) was dispensed into each well of a 96 well filter plate. Then 100 µl of affinity matrix (5% slurry) per well were added. The plate was incubated for two hours at 4°C on a shaker (750 rpm on a Thermomixer, Eppendorf). The plate was washed using a vacuum manifold instrument (Millipore, MAVM 096 0R). Each well was washed two times with 220 µl of 1x DP buffer containing 0.4% NP-40. For the elution of proteins the filter plate was placed on a collection plate and 20 µl of 2x sample buffer (100 mM TrisHCl, pH7.4; 4% SDS; 20% glycerol; 0.0002% Bromphenol blue) with DTT (50 mM final concentration) was added to each well. The plates were incubated for 30 minutes at room temperature on a shaker (750 rpm on a Thermomixer, Eppendorf). Subsequently the plates were centrifuged for four minutes at 1100 rpm (Heraeus centrifuge) and the eluate was collected in the wells of the collection plate.

### Detection and quantification of PI3Kdelta

The PI3Kdelta protein in the eluates was detected and quantified by spotting aliquots on a nitrocellulose membrane and detection with a first antibody directed against PI3Kdelta and a fluorescently labeled secondary antibody. The nitrocellulose membranes (BioTrace NT Nitrocellulose, PALL BTNT30R) were pretreated with 20% ethanol and subsequently washed with PBS buffer.

For detection of PI3Kdelta the membranes were first blocked by incubation with Odyssey blocking buffer (LICOR, 927-40000) for one hour at room temperature. Blocked membranes were then incubated for 16 hours at 4°C with the first antibody (anti PI3Kdelta, rabbit polyclonal antibody from Santa Cruz, catalogue number sc-7176) which was diluted 1:800 in Odyssey blocking buffer containing 0.2% Tween-20. After washing the membrane four times for seven minutes with PBS buffer containing 0.1% Tween 20 the membrane was incubated for 60 minutes with the detection antibody (goat ant-rabbit IRDye^{™} 800CW from LICOR, catalogue number 926-32211) diluted 1:2500 in Odyssey blocking buffer containing 0.2% Tween-20 and 0.02% SDS. Afterwards the membrane was washed four times for 5 minutes each with 1 x PBS buffer/0.1% Tween 20 and once for five minutes with 1 x PBS buffer. The membrane was kept in PBS buffer at 4°C and then scanned with the Odyssey instrument and signals were recorded and analysed according to the instructions of the manufacturer. The Odyssey Infrared Imaging system from LI-COR Biosciences (Lincoln, Nebraska, USA) was operated according to instructions provided by the manufacturer (Schutz-Geschwendener et al., 2004. Quantitative, two-color Western blot detection with infrared fluorescence. Published May 2004 by LI-COR Biosciences, www.licor.com).

### B) Compound profiling in cell lysates

### Preparation of cell lysates

The culture medium was removed, cells were washed once with D-PBS buffer and 4 ml ice-cold D-PBS buffer was added. Macrophages were removed by gently scraping the cells and resuspending them in D-PBS buffer. The cell suspensions were transferred into 15 ml Falcon tubes and kept on ice. The macrophage suspensions were centrifuged at 1500 rpm 4 °C for 3 minutes in a Heraeus Multifuge. The supernatant was removed and the cell pellets were washed with cold D-PBS buffer. After an additional centrifugation step, the cell pellets were quickly frozen in liquid nitrogen. Cells were thawed on ice and lysed by adding 120 µl 1x lysis buffer (1 x DP buffer, 0.8% NP40). The lysates were transferred into 1.5 ml Eppendorf tubes and incubated for 30 minutes rotating at 4 °C and then centrifuged for 10 minutes at 13,200 rpm at 4°C. The supernatants was transferred into ultracentrifuge tubes and centrifuged in a TLA-120.2 rotor at 53,000 rpm (100,000 x g) for 1 hour at 4°C. An aliquot of the clarified supernatant was used for protein quantification performing Bradford assay (Biorad Protein Assay dye concentrate, catalogue number 500-0006). The remaining samples were quickly frozen in liquid nitrogen and stored at -80°C until use in the binding assay.

### Dilution of cell lysate

Cell lysates were prepared as described below from RAW264.7 macrophages. One lysate aliquot was thawed in a 37°C water bath and then kept at 4°C. To the lysate one volume of 1xDP buffer containing protease inhibitor (1 tablet of protease inhibitor dissolved in 25 ml of 1x DP buffer or 25 ml of 1x DP buffer containing 0.8% NP40; EDTA-free tablet protease inhibitor cocktail from Roche Diagnostics, catalogue number 41647) was added so that a final NP40 concentration of 0.8% was achieved. The lysate was further diluted by adding 1x DP buffer containing 0.8% NP40 and proteinase inhibitors so that a final protein concentration of 10 mg/ml was achieved.

### Washing of affinity matrix

The affinity matrix as described in example 1 (0.25 ml of dry bead volume) was washed two times with 10 ml of 1x DP buffer containing 0.2% NP40 and was finally resuspended in 5.0 ml of 1x DP buffer containing 0.2% NP40 (5% beads slurry).

### Preparation of test compounds

For in the lysate competition experiment stock solutions of test compounds were prepared in DMSO corresponding to a 50fold higher concentration compared to the final concentration in the assay (for example a 500 µM stock solution was prepared for a final test concentration of 10 µM). This dilution scheme resulted in a final DMSO concentration of 2% in the assay. For control experiments (no test compound) a buffer containing 2% DMSO was used so that all test samples contained 2% DMSO.

### Incubation of cell lysate with test compound and affinity matrix

A volume of 50 µl of diluted lysate (10 mg/ml protein) was dispensed into each well of a 96 well filter plate. Then 3.0 µl of test compound diluted in DMSO was added. For control reactions 3.0 µl DMSO without test compound were used. Then 100 µl of affinity matrix (5% slurry) per well were added. The plate was incubated for two hours at 4°C on a shaker (750 rpm on a Thermomixer, Eppendorf). The plate was washed using a vacuum manifold instrument (Millipore, MAVM 096 0R). Each well was washed two times with 220 µl of 1x DP buffer containing 0.4% NP-40. For the elution of proteins the filter plate was placed on a collection plate and 20 µl of 2x sample buffer (100 mM TrisHCl, pH7.4; 4% SDS; 20% glycerol; 0.0002% Bromphenol blue) with DTT (50 mM final concentration) was added to each well. The plates were incubated for 30 minutes at room temperature on a shaker (750 rpm on a Thermomixer, Eppendorf). Subsequently the plates were centrifuged for four minutes at 1100 rpm (Heraeus centrifuge) and the eluate was collected in the wells of the collection plate. The detection and quantification of PI3Kdelta was performed as described above.

### Example 6: Selectivity profiling of PI3K interacting compounds using quantitative mass spectrometry

This examples demonstrates a competitive binding assay in which test compounds are added directly into a cell lysate. Test compounds (at various concentrations) and the affinity matrix (1:1 mixture of beads with immobilzed phenylthiazole ligand 1 and beads with immobilized phenylmorpholin-chromen ligand) were added to cell lysate aliquots and allowed to bind to the proteins contained in the lysate sample. After the incutation time the beads with captured proteins were separated from the lysate. Bound proteins were then eluted and the presence of kinases was measured using quantitative mass spectrometry based on the ITRAQ method. The IC₅₀ values for the interaction of three compounds with several kinase were determined (Figure 9).

### Washing of affinity matrix

The affinity matrix (1:1 mixture of beads with immobilzed phenylthiazole ligand 1 and beads with immobilized phenylmorpholin-chromen ligand) was washed two times with 15 ml of 1x DP buffer containing 0.4% NP40 and then resupended in 5.5 ml of 1x DP buffer containing 0.4% NP40 (20% beads slurry).

### Preparation of test compounds

Stock solutions of test compounds were prepared in DMSO corresponding to a 100fold higher concentration compared to the final desired test concentraion (e.g. a 4 mM stock solution was prepared for a final test concentration of 4 µM). This dilution scheme resulted in a final DMSO concentration of 1%. For control experiments (no test compound) a buffer containing 1% DMSO was used so that all test samples contained 1% DMSO.
Compound CZC00018052: dual PI3K/mTOR kinase inhibitor PI-103 (Calbiochem catalogue number 528100; Knight et al., 2006, Cell 125, 733-747).
Compound CZC00015097: PI3K gamma inhibitor I (Calbiochem 528106; AS-605240; Camps et al., 2005, Nature Medicine 11, 936-943).

### Dilution of cell lysate

Cell lysates were prepared from Ramos cells (ATCC number CRL-1596) as described in example 2. For a typical experiment one lysate aliquot containing 50 mg of protein was thawed in a 37°C water bath and then kept at 4°C. To the lysate one volume of 1xDP buffer was added so that a final NP40 concentration of 0.4% was achieved. Then, 1/50 of the final volume of a 50fold concentrated protease inhibitor solution was added (1 tablet of protease inhibitor dissolved in 0.5 ml of 1x DP buffer containing 0.4% NP40; EDTA-free tablet protease inhibitor cocktail from Roche Diagnostics, catalogue number 41647). The lysate was further dilute by adding 1x DP buffer containing 0.4% NP40 so that a final protein concentration of 5 mg/ml was achieved.

### Incubation of lysate with test compound and affinity matrix

A volume of 100 µl of diluted lysate was dispensed into each well of a 96 well filter plate. Then 1.5 µl of test compound diluted in DMSO was added. For control reactions 1.5 µl DMSO without test compound were used. Then 50 µl of affinity matrix (20% slurry) per well were added. The plate was incubated for 2 hours at 4°C on a shaker (750 rpm on a Thermomixer, Eppendorf).

The plate was washed using a vacuum manifold instrument (Millipore, MAVM 096 0R). Each well was washed 4 times with 400 µl of 1x DP buffer containing 0.4% NP-40 and 2 times with 400 µl with 1 x DP buffer containing 0.2% NP-40.

For elution the filter plate was placed on a collection plate and 40 µl of 2x sample buffer (100 mM TrisHCl, pH6.8; 4% SDS; 20% glycerol; 0.02% Bromphenol blue) with DTT (50 mM final concentration) was added to each well. The plates were incubated for 30 minutes at room temperature on a shaker (750 rpm on a Thermomixer, Eppendorf). Subsequently the plates were centrifuged for 2 minutes at 1100 rpm (Heraeus centrifuge) and the eluate was collected in the wells of the collection plate.

### Detection and quantification of kinases by mass spectrometry

The kinases in the eluate were detected by mass spectrometry as described in example 2 and quantitative analysis using the ITRAQ method was performed as described previously (WO 2006/134056; Bantscheff et al., 2007. Nature Biotechnology 25, 1035-1044) and IC₅₀ values were calculated for the interaction of individual compounds and kinases (Figure 9).

### Example 7: Selectivity profiling of PI3K interacting compounds using multiplex immunodetection

This examples demonstrates a competitive binding assay in which test compounds are added directly into a cell lysate. Test compounds (at various concentrations) and the affinity matrix (1:1 mixture of beads with immobilzed phenylthiazole ligand 1 and beads with immobilized phenylmorpholin-chromen ligand) were added to cell lysate aliquots and allowed to bind to the proteins contained in the lysate sample. After the incutation time the beads with captured proteins were separated from the lysate. Bound proteins were then eluted and the presence of kinases was detected and quantified using a multiplexed immunodetection format. Dose response curves for individual kinases were generated and IC₅₀ values calculated (Figures 10 and 11).

### Washing of affinity matrix

The affinity matrix (1:1 mixture of beads with immobilzed phenylthiazole ligand 1 and beads with immobilized phenylmorpholin-chromen ligand) was washed two times with 15 ml of 1x DP buffer containing 0.4% NP40 and then resupended in 5.5 ml of 1x DP buffer containing 0.4% NP40 (20% beads slurry).

### Preparation of test compounds

Stock solutions of test compounds were prepared in DMSO corresponding to a 100fold higher concentration compared to the final desired test concentraion (e.g. a 4 mM stock solution was prepared for a final test concentration of 4 µM). This dilution scheme resulted in a final DMSO concentration of 1%. For control experiments (no test compound) a buffer containing 1% DMSO was used so that all test samples contained 1% DMSO. Compound CZC00018052: dual PI3K/mTOR kinase inhibitor PI-103 (Calbiochem catalogue number 528100; Knight et al., 2006, Cell 125, 733-747).

### Dilution of cell lysate

Cell lysates were prepared as described in example 2. For this experiment a 1:1 mixture of Jurkat (ATCC catalogue number TIB-152 Jurkat, cloe E6-1) and Molt-4 (ATCC catalogue number CRL-1582) cell lysates was used. For a typical experiment one lysate aliquot containing 50 mg of protein was thawed in a 37°C water bath and then kept at 4°C. To the lysate one volume of 1xDP buffer was added so that a final NP40 concentration of 0.4% was achieved. Then, 1/50 of the final volume of a 50fold concentrated protease inhibitor solution was added (1 tablet of protease inhibitor dissolved in 0.5 ml of 1x DP buffer containing 0.4% NP40; EDTA-free tablet protease inhibitor cocktail from Roche Diagnostics, catalogue number 41647). The lysate was further dilute by adding 1x DP buffer containing 0.4% NP40 so that a final protein concentration of 5 mg/ml was achieved.

### Incubation of lysate with test compound and affinity matrix

To a 96 well filter plate (Multiscreen Solvinert Filter Plate, Millipore MSRL N04 10) were added: 100 µl affinity matrix (beads) per well, 3 µl of compound solution, and 50 µl of cell lysate. Plates were sealed and incubated for two hours in a cold room on a Thermoxer with shaking (750 rpm). Afterwards the plate was washed twice with 220 µl washing buffer. The beads were then eluted with 20 µl of sample buffer. The eluate was frozen qickly at -80°C and stored at -20°C.

### Detection and quantification of eluted kinases

The kinases in the eluates were detected and quantified by a spotting procedure on Nitrocellulose membranes using a first antibody directed against the kinase of interest and a fluorescently labeled secondary antibody (anti-mouse or anti-rabbit IRDye^{™} antibodies from Rockland). The Odyssey Infrared Imaging system from LI-COR Biosciences (Lincoln, Nebraska, USA) was operated according to instructions provided by the manufacturer (Schutz-Geschwendener et al., 2004. Quantitative, two-color Western blot detection with infrared fluorescence. Published May 2004 by LI-COR Biosciences, www.licor.com).

After spotting of the eluates the nitrocellulose membrane (BioTrace NT, Millipore #66485) was first blocked by incubation with Odyssey blocking buffer (LICOR, 927-40000) for one hour at room temperature. Blocked membranes were then incubated for 16 hours at 25°C with the first antibody which was diluted in Odyssey blocking buffer containing 0.2% Tween-20. Afterwards the membrane was washed four times for 7 minutes with PBS buffer containing 0.1% Tween 20. Then the membrane was incubated for 60 minutes at room temperature with the detection antibody (IRDye^{™} labelled antibody from Rockland) diluted in Odyssey blocking buffer containing 0.2% Tween-20 and 0.02% SDS. Afterwards the membrane was washed four times for 7 minutes each with 1 x PBS buffer/0.1% Tween 20 and once for 5 minutes with 1 x PBS buffer. The membrane was kept in PBS buffer at 4°C and then scanned with the Odyssey instrument. Fluorescence signals were recorded and analysed according to the instructions of the manufacturer.

Sources of antibodies:
Anti-PI3K gamma mouse (Jena Bioscience ABD-026); anti-PI3K delta (Santa Cruz #sc-7176); anti-PI3K alpha (Cell signaling #4255); anti-DNAPK (Calbiochem #NA57); Licor IRDye 800 mouse (926-32210); Licor IRDye 680 rabbit (926-32221); Licor IRDye 800 rabbit (926-32211); Licor IRDye 680 mouse (926-32220).

### Example 8: Preparation of the affinity matrix with the phenylmorpholin-chromen ligand

This example describes the synthesis of the phenylmorpholin-chromen ligand (8-(4-aminomethyl-phenyl)-2-morpholin-4-yl-chromen-4-one) (Figure 16). This capture ligand was immobilized on a solid support through covalent linkage using an amino functional group and used for the capturing of proteins from cell lysates (see for example Figure 17).

### Synthesis of 8-(4-aminomethyl-phenyl)-2-morpholin-4-yl-chromen-4-one

### Step 1

2,3-Dihydroxy-benzoic acid [A] (25g, 0.16mol) (Sigma-Aldrich, Cat no. 126209) was stirred in methanol (125ml) with concentrated sulphuric acid (1ml) and the reaction heated to gentle reflux over night. It was then concentrated and the residue partitioned between ethyl acetate and saturated aqueous sodium bicarbonate. The organic layer was washed with further saturated aqueous sodium bicarbonate, dried with magnesium sulphate, filtered and concentrated to afford 2,3-dihydroxy-benzoic acid methyl ester [B]. Yield 15.2g, 57%.
HPLC (Method B): (M-H⁺) 167; RT = 2.3min. ¹H NMR: (CDCl₃) δ 10.92 (s, 1H); 7.39 (dd, 1H); 7.13 (dd, 1H); 6.82 (dt, 1H); 3.70 (s, 1H); 3.98 (s, 3H).

### Step 2

2,3-Dihydroxy-benzoic acid methyl ester [B] (15.g, 89mmol) was dissolved in dichloromethane (100ml) with pyridine (3.6ml, 44.6mmol, 0.5eq) and DMAP (272mg, 2.2mmol, 0.025eq) and the reaction cooled in an ice/water bath. Trifluoromethanesulphonic anhydride (16.2ml, 98.2mmol, 1.1eq) was added, the reaction was allowed to warm to room temperature and stirred over night. The reaction mixture was diluted with dichloromethane, washed with 1M hydrochloric acid (150ml), dried with sodium sulphate, filtered and evaporated. The product was recrystallised from ethyl acetate to afford 2-hydroxy-3-trifluoromethanesulfonyloxy-benzoic acid methyl ester [C]. Yield crop 1, 6.5g, 24%. Further recrysatllisation afforded a second crop, 6.8g, 26%.
¹H NMR (CDCl₃): δ 11.11 (s, 1H); 7.80 (dd, 1H); 7.36 (dd, 1H); 6.86 (t, 1H); 3.93 (s, 3H).

### Step 3

A solution of *N*-acetylmorpholine (1.72g, 13.3mmol, 2eq) in 30ml dry tetrahydrofuran under nitrogen was cooled in an acetone/dry ice bath (-78°C) and treated with LDA (10ml, 2M solution in THF, 3eq). The reaction mixture was stirred for 60 minutes then 2-hydroxy-3-trifluoromethanesulfonyloxy-benzoic acid methyl ester [C] (2g, 6.6mmol, leq as a solution in 10ml dry THF) was added. The reaction mixture was allowed to warm from -78°C to room temperature and stirred over night. The reaction was diluted with water (4ml) followed by 2M hydrochloric acid (40ml), then extracted three times with dichloromethane. The extracts were combined, washed with brine, dried with magnesium sulphate, filtered and evaporated. The crude product was purified by flash chromatography eluting with ethyl acetate to afford trifluoro-methanesulfonic acid 2-hydroxy-3-(3-morpholin-4-yl-3-oxo-propionyl)-phenyl ester [D]. Yield 1.06g, 40%
¹H NMR (CDCl₃): δ 7.96 (dd, 1H); 7.49 (dd, 1H); 7.00 (t, 1H); 4.14 (s, 2H); 3.65-3.73 (m, 6H), 3.56 (t, 2H).

### Step 4

Trifluoro-methanesulfonic acid 2-hydroxy-3-(3-morpholin-4-yl-3-oxo-propionyl)-phenyl ester [D] (1.06g, 2.7mmol) in dichloromethane (30ml) was treated with trifluoromethanesulphonic anhydride and stirred over night at room temperature. The reaction mixture was then concentrated, re-dissolved in methanol and stirred for a further 2 hours. The solution was diluted with water and basified to pH8. It was then extracted three times with dichloromethane. The extracts were combined, washed with brine, dried with magnesium sulphate and evaporated to give the crude product as a brown oil. Trituration with ether gave trifluoro-methanesulfonic acid 2-morpholin-4-yl-4-oxo-4H-chromen-8-yl ester [E] as a brown solid. Yield 210mg, 20%.
HPLC (Method B): RT = 2.8min. ¹H NMR (CDCl₃): δ 8.16 (dd, 1H); 7.49 (dd, 1H); 7.40 (t, 1H); 5.62 (s, 1H); 3.85 (dd, 4H), 3.60 (dd, 4H).

### Step 5

Trifluoro-methanesulfonic acid 2-morpholin-4-yl-4-oxo-4H-chromen-8-yl ester [E] (380mg, 1.0mmol), 4-(*N*-Boc-aminomethyl)phenylboronic acid (280mg, 1.1mmol, 1.1eq), potassium carbonate (275mg, 2.0mmol, 2eq) and tetrakis triphenylphosphine palladium (0) (60mg, 0.05mmol 0.05eq) were stirred in dioxane (4ml) and heated to 80°C for 4 hours. The cooled reaction was then filtered and the filtrate concentrated *in vacuo.* The residue was purified by flash chromatography eluting with 0-3% methanol in dichloromethane to afford [4-(2-morpholin-4-yl-4-oxo-4H-chromen-8-yl)-benzyl]-carbamic acid tert-butyl ester [F]. Yield 238mg, 54%.
HPLC (Method A): (MH⁺) 437, (MNa⁺) 459; RT 3.0 min. ¹H NMR (CDCl₃) δ 8.17 (dd, 1H); 7.55 (dd, 1H); 7.49 (d, 2H); 7.37-7.42 (m, 3H); 5.51 (s, 1H), 5.00 (brs, 1H), 4.39 (d, 2H); 3.74 (dd, 4H); 3.35 (dd, 4H); 1.48 (s, 9H).

### Step 6

[4-(2-Morpholin-4-yl-4-oxo-4H-chromen-8-yl)-benzyl]-carbamic acid *tert-*butyl ester [F] (230mg, 0.53mmol), in dichloromethane (5ml) was treated with 4M hydrogen chloride in dioxane (2ml). The reaction was stirred at room temperature for 3 hours during which time a precipitate forms. The solvent was removed *in vacuo* and the residue triturated with ether. The resulting solid was collected by filtration and dried to give 8-(4-aminomethyl-phenyl)-2-morpholin-4-yl-chromen-4-one [G]. Yield 189mg, quantitative. HPLC (Method 18): (MH⁺) 337, (MNa⁺) 359; RT 1.32 min (broad). ¹H NMR (DMSO-d₆): δ 8.54 (brs, 2H); 7.99 (dd, 1H); 7.68-7.73 (m, 3H); 7.62 (d, 2H); 7.51 (t, 1H); 5.79 (s, 1H); 4.09 (q, 2H); 3.68 (t, 4H); 3.41 (t, 4H)

**Table 5: Abbreviations**

| | |
|---|---|
| DCM | Dichloromethane |
| DMAP | 4-(Dimethylamino)pyridine |
| LDA | Lithium diisopropylamide |
| MeOH | Methanol |
| THF | Tetrahydrofuran |

NMR spectra were obtained on a Bruker dpx400. LCMS was carried out on an Agilent 1100 using a ZORBAX^{®} SB-C18, 4.6 x 75 mm, 3.5 micron-column. Column flow was 1ml/min and solvents used were water and acetonitrile (0.1% formic acid) with an injection volume of 10ul. Wavelengths were 254 and 210nm. Methods are described below.

**Table 6: Analytical methods**

| **Method** | **Easy Access Method Name** | **ChemStation Method Name** | **Flow Rate** | **Solvent** | **Run Time** |
|---|---|---|---|---|---|
| A | Short column ANL Positive Medium | SANL_PGM.M | 1ml/min | 0-1.5min 30-95% MeCN | 5 min |
| | | | | 1.5-4.5 min 95% MeCN | |
| B | Short column ANL Negative Medium | SANL_NGM.M | 1ml/min | 0-1.5min 30-95% MeCN | 5 min |
| | | | | 1.5-4.5 min 95% MeCN | |

### Immobilization of the phenylmorpholin-chromen ligand on beads (affinity matrix)

NHS-activated Sepharose 4 Fast Flow (Amersham Biosciences, 17-0906-01) was equilibrated with anhydrous DMSO (Dimethylsulfoxid, Fluka, 41648, H20 <= 0.005%). 1 ml of settled beads was placed in a 15 ml Falcon tube, compound stock solution (usually 100 mM in DMF or DMSO) was added (final concentration 0.2-2 µmol/ml beads) as well as 15 µl of triethylamine (Sigma, T-0886, 99% pure). Beads were incubated at room temperature in darkness on an end-over-end shaker (Roto Shake Genie, Scientific Industries Inc.) for 16 - 20 hours. Coupling efficiency is determined by HPLC. Non-reacted NHS-groups were blocked by incubation with aminoethanol at room temperature on the end-over-end shaker over night. Beads were washed with 10 ml of DMSO and were stored in isopropanol at -20°C. These beads were used as the affinity matrix in example 2, 3 and 4. Control beads (no ligand immobilized) were generated by blocking the NHS-groups by incubation with aminoethanol as described above.

## Claims

1. A method for the identification of a PI3K interacting compound, comprising the steps of
a) providing a protein preparation containing PI3K,
b) contacting the protein preparation with 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide or salt thereof immobilized on a solid support under conditions allowing the formation of a 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide - PI3K complex,
c) incubating the 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide - PI3K complex with a given compound,
d) determining whether the compound is able to separate PI3K from the immobilized 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide, and
e) determining whether the compound is able to separate also ATM, ATR, DNAPK and/or mTOR from the immobilized 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide.

2. The method of claim 1, wherein step d) includes the detection of separated PINK or the determination of the amount of separated PI3K and/or wherein step e) includes the detection of separated ATM, ATR, DNAPK and/or mTOR or the determination of the amount of separated ATM, ATR, DNAPK and/or mTOR, preferably, wherein separated PI3K, ATM, ATR, DNAPK and/or mTOR is detected or the amount of separated PI3K, ATM, ATR, DNAPK and/or mTOR is determined by mass spectrometry or immunodetection methods, preferably with an antibody directed against PI3K, ATM, ATR, DNAPK and/or mTOR.

3. A method for the identification of a PI3K interacting compound, comprising the steps of
a) providing a protein preparation containing PI3K,
b) contacting the protein preparation with 3-(2-{2-[2-(2-amino-ethoxy)-ethoxy]-ethoxy} -ethoxy)-N-[5-(4-chloro-3-methanesulfonyl-phenyl)-4-methyl-thiazol-2yl]-propionamide or salt thereof immobilized on a solid support and with a given compound under conditions allowing the formation of a 3-(2-{2-[2-(2-amino-ethoxy)-ethoxy]-ethoxy} -ethoxy)-N-[5-(4-chloro-3-methanesulfonylphenyl)-4-methyl-thiazol-2yl]-propionamide - PI3K complex,
c) detecting the 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide-PI3K complex formed in step b), and
d) detecting whether also a complex between the 3-(2-{2-[2-(2-amino-ethoxy)-ethoxy]-ethoxy} -ethoxy)-N-[5-(4-chloro-3-methanesulfonyl-phenyl)-4-methyl-thiazol-2yl]-propionamide and ATM, ATR, DNAPK and or mTOR has been formed in step b).

4. The method of claim 3, wherein in step c) said detecting is performed by determining the amount of the 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide - PI3K complex and/or wherein in step d) the amount of a complex between the 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide and ATM, ATR, DNAPK and or mTOR is determined.

5. The method of any of claims 3 or 4, wherein steps a) to c) are performed with several protein preparations in order to test different compounds.

6. A method for the identification of a PI3K interacting compound, comprising the steps of:
a) providing two aliquots of a protein preparation containing PI3K,
b) contacting one aliquot with the 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide or salt thereof immobilized on a solid support under conditions allowing the formation of a 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide - PI3K complex,
c) contacting the other aliquot with the 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide or salt thereof immobilized on a solid support and with a given compound under conditions allowing the formation of a 3-(2-{2-[2-(2-amino-ethoxy)-ethoxy]-ethoxy} -ethoxy)-N-[5-(4-chloro-3-methanesulfonylphenyl)-4-methyl-thiazol-2yl]-propionamide - PI3K complex,
d) determining the amount of the 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide - PI3K complex formed in steps b) and c), and
e) determining whether also a complex between the 3-(2-{2-[2-(2-amino-ethoxy)-ethoxy]-ethoxy} -ethoxy)-N-[5-(4-chloro-3-methanesulfonyl-phenyl)-4-methyl-thiazol-2yl]-propionamide and ATM, ATR, DNAPK and or mTOR has been formed in steps b) and c).

7. A method for the identification of a PI3K interacting compound, comprising the steps of:
a) providing two aliquots comprising each at least one cell containing PI3K,
b) incubating one aliquot with a given compound,
c) harvesting the cells of each aliquot,
d) lysing the cells in order to obtain protein preparations,
e) contacting the protein preparations with the 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide or salt thereof immobilized on a solid support under conditions allowing the formation of a 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide - PI3K complex, and
f) determining the amount of the 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide - PI3K complex formed in each aliquot in step e), and
g) determining whether also a complex between the 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamid and ATM, ATR, DNAPK and or mTOR has been formed in step e).

8. The method of any of claims 6 or 7, wherein a reduced amount of the 3-(2-{2-[2-(2-amino-ethoxy)-ethoxy]-ethoxy}-ethoxy)-N-[5-(4-chloro-3-methanesulfonylphenyl)-4-methyl-thiazol-2yl]-propionamide - PI3K complex formed in the aliquot incubated with the compound in comparison to the aliquot not incubated with the compound indicates that PI3K is a target of the compound.

9. The method of any of claims 4 to 8, wherein the amount of the 3-(2-{2-[2-(2-amino-ethoxy)-ethoxy]-ethoxy}-ethoxy)-N-[5-(4-chloro-3-methanesulfonylphenyl)-4-methyl-thiazol-2yl]-propionamide - PI3K complex is determined by separating PI3K from the immobilized 3-(2-{2-[2-(2-amino-ethoxy)-ethoxy]-ethoxy} -ethoxy)-N-[5-(4-chloro-3-methanesulfonyl-phenyl)-4-methyl-thiazol-2yl]-propionamide and subsequent detection of separated PI3K or subsequent determination of the amount of separated PI3K.

10. The method of any of claims 4 to 9, wherein said determination whether also a complex between 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide and ATM, ATR, DNAPK and/or mTOR has been formed is performed by separating said protein from the immobilized 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide and subsequent detection of separated ATM, ATR, DNAPK and or mTOR or subsequent determination of the amount of separated ATM, ATR, DNAPK and or mTOR, preferably wherein said protein is detected or the amount of said protein is determined by mass spectrometry or immunodetection methods, preferably with an antibody directed against said protein.

11. The method of any of claims 1 to 10, performed as a medium or high throughput screening, and/or wherein said compound is selected from the group consisting of synthetic compounds, or organic synthetic drugs, more preferably small molecule organic drugs, and natural small molecule compounds.

12. The method of any of claims 1 to 11, wherein the PI3K interacting compound is a PI3K inhibitor, and/or wherein the solid support is selected from the group consisting of agarose, modified agarose, sepharose beads (e.g. NHS-activated sepharose), latex, cellulose, and ferro- or ferrimagnetic particles, and/or wherein the 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide is covalently coupled to the solid support.

13. The method of any of claims 1 to 12, wherein the PI3K is PI3K gamma and/or PI3K delta.

14. The method of any of claims 1 to 13, wherein the provision of a protein preparation includes the steps of harvesting at least one cell containing PI3K and lysing the cell.

15. The method of any of claims 1 to 14, wherein the steps of the formation of the 3-(2-{2-[2-(2-amino-ethoxy)-ethoxy]-ethoxy}-ethoxy)-N-[5-(4-chloro-3-methanesulfonyl-phenyl)-4-methyl-thiazol-2yl]-propionamide - PI3K complex are performed under essentially physiological conditions.

## Patentansprüche

1. Verfahren für die Identifizierung einer PI3K-interagierenden Verbindung, umfassend die Schritte:
a) Bereitstellen einer Proteinaufbereitung enthaltend PI3K,
b) In-Kontakt-Bringen der Proteinaufbereitung mit 3-(2-{2-[2-(2-Amino-ethoxy)-ethoxy]-ethoxy}-ethoxy)-N-[5-(4-chlor-3-methansulfonyl-phenyl)-4-methyl-thiazol-2yl]-propionamid oder einem Salz davon, immobilisiert auf einem festen Träger unter Bedingungen, die die Bildung eines 3-(2-{2-[2-(2-Amino-ethoxy)-ethoxy]-ethoxy}-ethoxy)-N-[5-(4-chlor-3-methansulfonyl-phenyl)-4-methyl-thiazol-2yl]-propionamid-PI3K-Komplexes erlauben,
c) Inkubieren des 3-(2-{2-[2-(2-Amino-ethoxy)-ethoxy]-ethoxy}-ethoxy)-N-[5-(4-chlor-3-methansulfonyl-phenyl)-4-methyl-thiazol-2yl]-propionamid-PI3K-Komplexes mit einer gegebenen Verbindung,
d) Bestimmen, ob die Verbindung in der Lage ist, PI3K von dem immobilisierten 3-(2-{2-[2-(2-Amino-ethoxy)-ethoxy]-ethoxy}-ethoxy)-N-[5-(4-chlor-3-methansulfonyl-phenyl)-4-methyl-thiazol-2yl]-propionamid abzutrennen, und
e) Bestimmen, ob die Verbindung in der Lage ist, ebenso ATM, ATR, DNAPK und/oder mTOR von dem immobilisierten 3-(2-{2-[2-(2-Amino-ethoxy)-ethoxy]-ethoxy}-ethoxy)-N-[5-(4-chlor-3-methansulfonyl-phenyl)-4-methyl-thiazol-2yl]-propionamid abzutrennen.

2. Verfahren nach Anspruch 1, wobei Schritt d) die Detektion von abgetrennter PI3K oder die Bestimmung der Menge an abgetrennter PI3K einschließt und/oder wobei Schritt e) die Detektion von abgetrennter ATM, ATR, DNAPK und/oder mTOR oder die Bestimmung der Menge an abgetrennter ATM, ATR, DNAPK und/oder mTOR einschließt, vorzugsweise wobei abgetrennte PI3K, ATM, ATR, DNAPK und/oder mTOR detektiert wird oder die Menge an abgetrennter PI3K, ATM, ATR, DNAPK und/oder mTOR mittels Massenspektrometrie oder Immundetektionsverfahren bestimmt wird, vorzugsweise mit einem Antikörper gerichtet gegen PI3K, ATM, ATR, DNAPK und/oder mTOR.

3. Verfahren für die Identifizierung einer PI3K-interagierenden Verbindung, umfassend die Schritte:
a) Bereitstellen einer Proteinaufbereitung enthaltend PI3K,
b) In-Kontakt-Bringen der Proteinaufbereitung mit 3-(2-{2-[2-(2-Amino-ethoxy)-ethoxy]-ethoxy}-ethoxy)-N-[5-(4-chlor-3-methansulfonyl-phenyl)-4-methyl-thiazol-2yl]-propionamid oder einem Salz davon, immobilisiert auf einem festen Träger und mit einer gegebenen Verbindung unter Bedingungen, die die Bildung eines 3-(2-{2-[2-(2-Amino-ethoxy)-ethoxy]-ethoxy}-ethoxy)-N-[5-(4-chlor-3-methansulfonyl-phenyl)-4-methyl-thiazol-2yl]-propionamid-PI3K-Komplexes erlauben,
c) Detektieren des in Schritt b) gebildeten 3-(2-{2-[2-(2-Amino-ethoxy)-ethoxy]-ethoxy}-ethoxy)-N-[5-(4-chlor-3-methansulfonyl-phenyl)-4-methyl-thiazol-2yl]-propionamid-PI3K-Komplexes, und
d) Detektieren, ob sich ebenfalls ein Komplex zwischen dem 3-(2-{2-[2-(2-Amino-ethoxy)-ethoxy]-ethoxy}-ethoxy)-N-[5-(4-chlor-3-methansulfonyl-phenyl)-4-methyl-thiazol-2yl]-propionamid und ATM, ATR, DNAPK und/oder mTOR in Schritt b) gebildet hat.

4. Verfahren nach Anspruch 3, wobei in Schritt c) das Detektieren durch Bestimmen der Menge an 3-(2-{2-[2-(2-Amino-ethoxy)-ethoxy]-ethoxy}-ethoxy)-N-[5-(4-chlor-3-methansulfonyl-phenyl)-4-methyl-thiazol-2yl]-propionamid-PI3K-Komplex erfolgt und/oder wobei in Schritt d) die Menge eines Komplexes zwischen dem 3-(2-{2-[2-(2-Amino-ethoxy)-ethoxy]-ethoxy}-ethoxy)-N-[5-(4-chlor-3-methansulfonyl-phenyl)-4-methyl-thiazol-2yl]-propionamid und ATM, ATR, DNAPK und/oder mTOR bestimmt wird.

5. Verfahren nach einem beliebigen der Ansprüche 3 oder 4, wobei die Schritte a) bis c) mit verschiedenen Proteinaufbereitungen durchgeführt werden, um verschiedene Verbindungen zu testen.

6. Verfahren für die Identifizierung einer PI3K-interagierenden Verbindung, umfassend die Schritte:
a) Bereitstellen zweier Aliquote einer Proteinaufbereitung enthaltend PI3K,
b) In-Kontakt-Bringen eines Aliquots mit dem auf einem festen Träger immobilisierten 3-(2-{2-[2-(2-Amino-ethoxy)-ethoxy]-ethoxy}-ethoxy)-N-[5-(4-chlor-3-methansulfonyl-phenyl)-4-methyl-thiazol-2yl]-propionamid oder einem Salz davon unter Bedingungen, die die Bildung eines 3-(2-{2-[2-(2-Amino-ethoxy)-ethoxy]-ethoxy}-ethoxy)-N-[5-(4-chlor-3-methansulfonyl-phenyl)-4-methyl-thiazol-2yl]-propionamid-PI3K-Komplexes erlauben,
c) In-Kontakt-Bringen des anderen Aliquots mit dem auf einem festen Träger immobilisierten 3-(2-{2-[2-(2-Amino-ethoxy)-ethoxy]-ethoxy}-ethoxy)-N-[5-(4-chlor-3-methansulfonyl-phenyl)-4-methyl-thiazol-2yl]-propionamid oder einem Salz davon und mit einer gegebenen Verbindung unter Bedingungen, die die Bildung eines 3-(2-{2-[2-(2-Amino-ethoxy)-ethoxy]-ethoxy}-ethoxy)-N-[5-(4-chlor-3-methansulfonyl-phenyl)-4-methyl-thiazol-2yl]-propionamid-PI3K-Komplexes erlauben,
d) Bestimmen der Menge des in den Schritten b) und c) gebildeten 3-(2-{2-[2-(2-Amino-ethoxy)-ethoxy]-ethoxy}-ethoxy)-N-[5-(4-chlor-3-methansulfonyl-phenyl)-4-methyl-thiazol-2yl]-propionamid-PI3K-Komplexes, und
e) Bestimmen, ob sich ebenfalls ein Komplex zwischen dem 3-(2-{2-[2-(2-Amino-ethoxy)-ethoxy]-ethoxy}-ethoxy)-N-[5-(4-chlor-3-methansulfonyl-phenyl)-4-methyl-thiazol-2yl]-propionamid und ATM, ATR, DNAPK und/oder mTOR in den Schritten b) und c) gebildet hat.

7. Verfahren für die Identifizierung einer PI3K-interagierenden Verbindung, umfassend die Schritte:
a) Bereitstellen zweier Aliquots, umfassend jeweils mindestens eine Zelle, die PI3K enthält,
b) Inkubieren eines Aliquots mit einer gegebenen Verbindung,
c) Ernten der Zellen eines jeden Aliquots,
d) Lysieren der Zellen, um Proteinaufbereitungen zu erhalten,
e) In-Kontakt-Bringen der Proteinaufbereitungen mit dem auf einem festen Träger immobilisierten 3-(2-{2-[2-(2-Amino-ethoxy)-ethoxy]-ethoxy}-ethoxy)-N-[5-(4-chlor-3-methansulfonyl-phenyl)-4-methyl-thiazol-2yl]-propionamid oder einem Salz davon unter Bedingungen, die die Bildung eines 3-(2-{2-[2-(2-Amino-ethoxy)-ethoxy]-ethoxy}-ethoxy)-N-[5-(4-chlor-3-methansulfonyl-phenyl)-4-methyl-thiazol-2yl]-propionamid-PI3K-Komplexes erlauben, und
f) Bestimmen der Menge des 3-(2-{2-[2-(2-Amino-ethoxy)-ethoxy]-ethoxy}-ethoxy)-N-[5-(4-chlor-3-methansulfonyl-phenyl)-4-methyl-thiazol-2yl]-propionamid-PI3K-Komplexes, die sich in Schritt e) in jedem Aliquot gebildet hat, und
g) Bestimmen, ob sich ebenfalls ein Komplex zwischen dem 3-(2-{2-[2-(2-Amino-ethoxy)-ethoxy]-ethoxy}-ethoxy)-N-[5-(4-chlor-3-methansulfonyl-phenyl)-4-methyl-thiazol-2yl]-propionamid und ATM, ATR, DNAPK und/oder mTOR in Schritt e) gebildet hat.

8. Verfahren nach einem beliebigen der Ansprüche 6 oder 7, wobei eine reduzierte Menge des 3-(2-{2-[2-(2-Amino-ethoxy)-ethoxy]-ethoxy}-ethoxy)-N-[5-(4-chlor-3-methansulfonyl-phenyl)-4-methyl-thiazol-2yl]-propionamid-PI3K-Komplexes, welcher sich in dem mit der Verbindung inkubierten Aliquot im Vergleich zu dem Aliquot, welches nicht mit der Verbindung inkubiert wurde, gebildet hat, ein Hinweis darauf ist, dass PI3K ein Ziel dieser Verbindung ist.

9. Verfahren nach einem beliebigen der Ansprüche 4 bis 8, wobei die Menge an 3-(2-{2-[2-(2-Amino-ethoxy)-ethoxy]-ethoxy}-ethoxy)-N-[5-(4-chlor-3-methansulfonyl-phenyl)-4-methyl-thiazol-2yl]-propionamid-PI3K-Komplex durch Abtrennen der PI3K von dem immobilisierten 3-(2-{2-[2-(2-Amino-ethoxy)-ethoxy]-ethoxy}-ethoxy)-N-[5-(4-chlor-3-methansulfonyl-phenyl)-4-methyl-thiazol-2yl]-propionamid und anschließender Detektion der abgetrennten PI3K oder anschließender Bestimmung der Menge an abgetrennter PI3K bestimmt wird.

10. Verfahren nach einem beliebigen der Ansprüche 4 bis 9, wobei die Bestimmung, ob sich ebenfalls ein Komplex zwischen 3-(2-{2-[2-(2-Amino-ethoxy)-ethoxy]-ethoxy}-ethoxy)-N-[5-(4-chlor-3-methansulfonyl-phenyl)-4-methyl-thiazol-2yl]-propionamid und ATM, ATR, DNAPK und/oder mTOR gebildet hat, durch Abtrennen des Proteins von dem immobilisierten 3-(2-{2-[2-(2-Amino-ethoxy)-ethoxy]-ethoxy}-ethoxy)-N-[5-(4-chlor-3-methansulfonyl-phenyl)-4-methyl-thiazol-2yl]-propionamid und anschließender Detektion der abgetrennten ATM, ATR, DNAPK und/oder mTOR oder anschließender Bestimmung der Menge an abgetrennter ATM, ATR, DNAPK und/oder mTOR durchgeführt, vorzugsweise wobei das Protein detektiert wird oder die Menge des Proteins mittels Massenspektrometrie oder Immundetektionsverfahren bestimmt wird, vorzugsweise mit einem Antikörper gerichtet gegen das Protein.

11. Verfahren nach einem beliebigen der Ansprüche 1 bis 10, durchgeführt als ein Mittel- oder Hochdurchsatzsscreening und/oder wobei die Verbindung ausgewählt ist aus der Gruppe bestehend aus synthetischen Verbindungen oder organischen synthetischen Wirkstoffen, stärker bevorzugt niedermolekularen organischen Wirkstoffen und natürlichen niedermolekularen Verbindungen.

12. Verfahren nach einem beliebigen der Ansprüche 1 bis 11, wobei die PI3K-interagierende Verbindung ein PI3K-Inhibitor ist, und/oder wobei der feste Träger ausgewählt ist aus der Gruppe bestehend aus Agarose, modifizierter Agarose, Sepharose-Harzkörner (z.B. NHS-aktivierte Sepharose), Latex, Zellulose und ferro-oder ferrimagnetischen Partikeln und/oder wobei das 3-(2-{2-[2-(2-Amino-ethoxy)-ethoxy]-ethoxy}-ethoxy)-N-[5-(4-chlor-3-methansulfonyl-phenyl)-4-methyl-thiazol-2yl]-propionamid kovalent an den festen Träger gekoppelt ist.

13. Verfahren nach einem beliebigen der Ansprüche 1 bis 12, wobei die PI3K PI3K-gamma und/oder PI3K-delta ist.

14. Verfahren nach einem beliebigen der Ansprüche 1 bis 13, wobei die Bereitstellung einer Proteinaufbereitung die Schritte des Ernten mindestens einer PI3K-enthaltenden Zelle und Lysieren der Zelle einschließt.

15. Verfahren nach einem beliebigen der Ansprüche 1 bis 14, wobei die Schritte der Bildung des 3-(2-{2-[2-(2-Amino-ethoxy)-ethoxy]-ethoxy}-ethoxy)-N-[5-(4-chlor-3-methansulfonyl-phenyl)-4-methyl-thiazol-2yl]-propionamid-PI3K-Komplexes unter im Wesentlichen physiologischen Bedingungen durchgeführt werden.

## Revendications

1. Procédé d'identification d'un composé interagissant avec la P13K, comprenant les étapes de
a) fourniture d'une préparation de protéine contenant de la P13K,
b) mise en contact de la préparation de protéine avec du 3-(2-{2-[2-(2-amino-éthoxy)-éthoxy]-éthoxy}-éthoxy)-N-[5-(4-chloro-3-méthanesulfonylphényl)-4-méthylthiazol-2-yl]-propionamide ou avec un sel de celui-ci, immobilisé sur un support solide, dans des conditions permettant la formation d'un complexe de 3-(2-{2-[2-(2-amino-éthoxy)-éthoxy]-éthoxy}-éthoxy)-N-[5-(4-chloro-3-méthanesulfonyl-phényl)-4-méthylthiazol-2-yl]-propionamide - P13K,
c) incubation du complexe de 3-(2-{2-[2-(2-amino-éthoxy)-éthoxy]-éthoxy}-éthoxy)-N-[5-(4-chloro-3-méthanesulfonyl-phényl)-4-méthylthiazol-2-yl]-propionamide - P13K avec un composé donné,
d) détermination de la capacité du composé à séparer la P13K du 3-(2-{2-[2-(2-amino-éthoxy)-éthoxy]-éthoxy}-éthoxy)-N-[5-(4-chloro-3-méthanesulfonyl-phényl)-4-méthylthiazol-2-yl]-propionamide immobilisé, et
e) détermination de la capacité du composé à séparer également l'ATM, l'ATR, la DNAPK et/ou la mTOR du 3-(2-{2-[2-(2-amino-éthoxy)-éthoxy]-éthoxy}-éthoxy)-N-[5-(4-chloro-3-méthanesulfonyl-phényl)-4-méthylthiazol-2-yl]-propionamide immobilisé.

2. Procédé selon la revendication 1, dans lequel l'étape d) comprend la détection de la P13K séparée ou la détermination de la quantité de P13K séparée et/ou dans lequel l'étape e) comprend la détection de l'ATM, de l'ATR, de la DNAPK et/ou de la mTOR séparées ou la détermination de la quantité d'ATM, d'ATR, de DNAPK et/ou de mTOR séparées, de préférence dans lequel la P13K, l'ATM, l'ATR, la DNAPK et/ou la mTOR séparées sont détectés ou la quantité de P13K, d'ATM, d'ATR, de DNAPK et/ou de mTOR séparées est déterminée par des procédés de spectrométrie de masse ou d'immunodétection, de préférence avec un anticorps dirigé contre la P13K, l'ATM, l'ATR, la DNAPK et/ou la mTOR.

3. Procédé d'identification d'un composé interagissant avec la P13K, comprenant les étapes de
a) fourniture d'une préparation de protéine contenant de la P13K,
b) mise en contact de la préparation de protéine avec du 3-(2-{2-[2-(2-amino-éthoxy)-éthoxy]-éthoxy}-éthoxy)-N-[5-(4-chloro-3-méthanesulfonylphényl)-4-méthylthiazol-2-yl]-propionamide ou avec un sel de celui-ci, immobilisé sur un support solide, et avec un composé donné dans des conditions permettant la formation d'un complexe de 3-(2-{2-[2-(2-amino-éthoxy)-éthoxy]-éthoxy}-éthoxy)-N-[5-(4-chloro-3-méthanesulfonyl-phényl)-4-méthylthiazol-2-yl]-propionamide - P13K,
c) détection du complexe de 3-(2-{2-[2-(2-amino-éthoxy)-éthoxy]-éthoxy}-éthoxy)-N-[5-(4-chloro-3-méthanesulfonyl-phényl)-4-méthylthiazol-2-yl]-propionamide - P13K formé dans l'étape b), et
d) détection également si un complexe entre le 3-(2-{2-[2-(2-amino-éthoxy)-éthoxy]-éthoxy}-éthoxy)-N-[5-(4-chloro-3-méthanesulfonyl-phényl)-4-méthylthiazol-2-yl]-propionamide et de l'ATM, de l'ATR, de la DNAPK et/ou de la mTOR a été formé dans l'étape b).

4. Procédé selon la revendication 3, dans lequel dans l'étape c), ladite détection est réalisée par détermination de la quantité de complexe 3-(2-{2-[2-(2-amino-éthoxy)-éthoxy]-éthoxy}-éthoxy)-N-[5-(4-chloro-3-méthanesulfonyl-phényl)-4-méthylthiazol-2-yl]-propionamide - P13K et/ou dans lequel dans l'étape d), la quantité de complexe entre le 3-(2-{2-[2-(2-amino-éthoxy)-éthoxy]-éthoxy}-éthoxy)-N-[5-(4-chloro-3-méthanesulfonyl-phényl)-4-méthylthiazol-2-yl]-propionamide et l'ATM, l'ATR, la DNAPK et/ou la mTOR est déterminée.

5. Procédé selon l'une quelconque des revendications 3 ou 4, dans lequel les étapes a) à c) sont réalisées avec plusieurs préparations de protéine afin de tester différents composés.

6. Procédé d'identification d'un composé interagissant avec la P13K, comprenant les étapes de
a) fourniture de deux aliquotes d'une préparation de protéine contenant de la P13K,
b) mise en contact d'une aliquote avec du 3-(2-{2-[2-(2-amino-éthoxy)-éthoxy]-éthoxy}-éthoxy)-N-[5-(4-chloro-3-méthanesulfonylphényl)-4-méthylthiazol-2-yl]-propionamide ou avec un sel de celui-ci, immobilisé sur un support solide, dans des conditions permettant la formation d'un complexe de 3-(2-{2-[2-(2-amino-éthoxy)-éthoxy]-éthoxy}-éthoxy)-N-[5-(4-chloro-3-méthanesulfonyl-phényl)-4-méthylthiazol-2-yl]-propionamide-P13K,
c) mise en contact de l'autre aliquote avec du 3-(2-{2-[2-(2-amino-éthoxy)-éthoxy]-éthoxy}-éthoxy)-N-[5-(4-chloro-3-méthanesulfonylphényl)-4-méthylthiazol-2-yl]-propionamide ou avec un sel de celui-ci, immobilisé sur un support solide, et avec un composé donné, dans des conditions permettant la formation d'un complexe de 3-(2-{2-[2-(2-amino-éthoxy)-éthoxy]-éthoxy}-éthoxy)-N-[5-(4-chloro-3-méthanesulfonyl-phényl)-4-méthylthiazol-2-yl]-propionamide - P13K,
d) détermination de la quantité de complexe de 3-(2-{2-[2-(2-amino-éthoxy)-éthoxy]-éthoxy}-éthoxy)-N-[5-(4-chloro-3-méthanesulfonyl-phényl)-4-méthylthiazol-2-yl]-propionamide - P13K formé dans les étapes b) et c), et
e) détermination également si un complexe entre le 3-(2-{2-[2-(2-amino-éthoxy)-éthoxy]-éthoxy}-éthoxy)-N-[5-(4-chloro-3-méthanesulfonyl-phényl)-4-méthylthiazol-2-yl]-propionamide et l'ATM, l'ATR, la DNAPK et/ou la mTOR a été formé dans les étapes b) et c).

7. Procédé d'identification d'un composé interagissant avec la P13K, comprenant les étapes de
a) fourniture de deux aliquotes comprenant chacune au moins une cellule contenant de la P13K,
b) incubation d'une aliquote avec un composé donné,
c) récolte des cellules de chaque aliquote,
d) lyse des cellules afin d'obtenir des préparations de protéine,
e) mise en contact des préparations de protéine avec du 3-(2-{2-[2-(2-amino-éthoxy)-éthoxy]-éthoxy}-éthoxy)-N-[5-(4-chloro-3-méthanesulfonylphényl)-4-méthylthiazol-2-yl]-propionamide ou avec un sel de celui-ci, immobilisé sur un support solide, dans des conditions permettant la formation d'un complexe de 3-(2-{2-[2-(2-amino-éthoxy)-éthoxy]-éthoxy}-éthoxy)-N-[5-(4-chloro-3-méthanesulfonyl-phényl)-4-méthylthiazol-2-yl]-propionamide - P13K, et
f) détermination de la quantité de complexe de 3-(2-{2-[2-(2-amino-éthoxy)-éthoxy]-éthoxy}-éthoxy)-N-[5-(4-chloro-3-méthanesulfonyl-phényl)-4-méthylthiazol-2-yl]-propionamide - P13K formé dans chaque aliquote dans l'étape e), et
g) détermination également si un complexe entre le 3-(2-{2-[2-(2-amino-éthoxy)-éthoxy]-éthoxy}-éthoxy)-N-[5-(4-chloro-3-méthanesulfonyl-phényl)-4-méthylthiazol-2-yl]-propionamide et l'ATM, l'ATR, la DNAPK et/ou la mTOR a été formé dans l'étape e).

8. Procédé selon l'une quelconque des revendications 6 ou 7, dans lequel une quantité réduite de complexe de 3-(2-{2-[2-(2-amino-éthoxy)-éthoxy]-éthoxy}-éthoxy)-N-[5-(4-chloro-3-méthanesulfonyl-phényl)-4-méthylthiazol-2-yl]-propionamide - P13K formé dans l'aliquote incubée avec le composé par rapport à l'aliquote non incubée avec le composé indique que la P13K est une cible du composé.

9. Procédé selon l'une quelconque des revendications 4 à 8, dans lequel la quantité de complexe de 3-(2-{2-[2-(2-amino-éthoxy)-éthoxy]-éthoxy}-éthoxy)-N-[5-(4-chloro-3-méthanesulfonyl-phényl)-4-méthylthiazol-2-yl]-propionamide - P13K est déterminée par séparation de la P13K du 3-(2-{2-[2-(2-amino-éthoxy)-éthoxy]-éthoxy}-éthoxy)-N-[5-(4-chloro-3-méthanesulfonyl-phényl)-4-méthylthiazol-2-yl]-propionamide immobilisé et ensuite par détection de la P13K séparée ou par détermination ultérieure de la quantité de P13K séparée.

10. Procédé selon l'une quelconque des revendications 4 à 9, dans lequel ladite détermination du fait qu'un complexe entre le 3-(2-{2-[2-(2-amino-éthoxy)-éthoxy]-éthoxy}-éthoxy)-N-[5-(4-chloro-3-méthanesulfonyl-phényl)-4-méthylthiazol-2-yl]-propionamide et l'ATM, l'ATR, la DNAPK et/ou la mTOR a été formé est réalisée par séparation de ladite protéine du 3-(2-{2-[2-(2-amino-éthoxy)-éthoxy]-éthoxy}-éthoxy)-N-[5-(4-chloro-3-méthanesulfonyl-phényl)-4-méthylthiazol-2-yl]-propionamide immobilisé et ensuite par détection de l'ATM, de l'ATR, de la DNAPK et/ou de la mTOR séparées ou par détermination ultérieure de la quantité d'ATM, d'ATR, de DNAPK et/ou de mTOR séparées, de préférence dans lequel ladite protéine est détectée ou la quantité de ladite protéine est déterminée par des procédés de spectrométrie de masse ou d'immunodétection, de préférence avec un anticorps dirigé contre ladite protéine.

11. Procédé selon l'une quelconque des revendications 1 à 10, mis en oeuvre en criblage à moyen ou haut débit et/ou dans lequel ledit composé est choisi dans le groupe constitué par les composés synthétiques ou les médicaments organiques synthétiques, plus préférentiellement les médicaments de type petite molécule organique et les petites molécules naturelles.

12. Procédé selon l'une quelconque des revendications 1 à 11, dans lequel le composé interagissant avec la P13K est un inhibiteur de P13K, et/ou dans lequel le support solide est choisi dans le groupe constitué par l'agarose, l'agarose modifié, les billes de sépharose (par exemple la sépharose activée par du NHS), le latex, la cellulose et les particules ferro- ou ferri-magnétiques, et/ou dans lequel le 3-(2-{2-[2-(2-amino-éthoxy)-éthoxy]-éthoxy}-éthoxy)-N-[5-(4-chloro-3-méthanesulfonyl-phényl)-4-méthylthiazol-2-yl]-propionamide est couplé par covalence avec le support solide.

13. Procédé selon l'une quelconque des revendications 1 à 12, dans lequel la P13K est la P13K gamma et/ou la P13K delta.

14. Procédé selon l'une quelconque des revendications 1 à 13, dans lequel la fourniture d'une préparation de protéine comprend les étapes de récolte d'au moins une cellule contenant de la P13K et de lyse de la cellule.

15. Procédé selon l'une quelconque des revendications 1 à 14, dans lequel les étapes de formation du complexe de 3-(2-{2-[2-(2-amino-éthoxy)-éthoxy]-éthoxy]-éthoxy)-N-[5-(4-chloro-3-méthanesulfonyl-phényl)-4-méthylthiazol-2-yl]-propionamide - P13K sont réalisées dans des conditions essentiellement physiologiques.
